# EUROPEAN PATENT APPLICATION

(11) **EP 4 317 987 A1**
(43) Date of publication of application: **07.02.2024**
(21) Application number: 22775799.4
(22) Date of filing: 24.03.2022
(51) Int. Cl.: G01N 35/00, G01N 33/543

(54) **IMMUNOCHROMATOGRAPHIC TESTING DEVICE**

(30) Priority: 24.03.2021 JP 2021050780
(71) Applicant: FUJIFILM Corporation, Tokyo 106-8620 (JP)
(72) Inventor: TANAKA, Yasutake, Ashigarakami-gun, Kanagawa 258-8538 (JP)
(74) Representative: Hughes, Andrea Michelle
(86) International application number: PCT/JP2022/014115
(87) International publication number: WO 2022/203018

(57) **Abstract**

An assay apparatus includes a loading part in which a cartridge including a carrier having a spotting region on which a sample is spotted and an assay region is attachably and detachably loaded, an imaging unit that images the assay region, and a processor configured to perform a main determination, which is a determination of whether the sample is positive or negative, based on an assay region image imaged by the imaging unit. The assay region image is an image in which a plurality of pixels are two-dimensionally arranged in a matrix form, and the processor is configured to perform an abnormality determination for determining presence or absence of an abnormality in the assay region image, using, as a determination index, at least one of a difference between a relatively large pixel value and a relatively small pixel value in at least one column in the assay region image, a standard deviation based on the pixel values in each of the columns, or a variation coefficient based on the pixel values in each of the columns, and determine the processing content related to the main determination based on the presence or absence of the abnormality.

## Description

### BACKGROUND OF THE INVENTION

### 1. Field of the Invention

The present disclosure relates to an immunochromatographic assay apparatus.

### 2. Description of the Related Art

Among immunoassay methods, an immunochromatographic method is generally widely utilized, because operation is easy and assay can be performed within a short period of time.

In the immunochromatographic method, an immunochromatographic carrier provided with an assay region on which an antibody that specifically binds to an antigen, which is a test substance, is immobilized is used. In a case where a labeled antibody that specifically binds to an antigen is developed on an immunochromatographic carrier together with a sample containing the antigen, the antigen binds to the antibody immobilized on the assay region and the labeling substance is captured via the antigen. Since the assay region develops a color from the labeling substance captured in the assay region, the sample is determined as positive in a case where the color optical density of the assay region is not less than the reference value.

JP2009-115470A discloses an assay apparatus that optically analyzes a color development state of an assay region of an immunochromatographic carrier. The assay apparatus includes a sensor that optically detects a reaction state and a measurement unit that determines whether the sample is positive or negative based on the detection result. In JP2009-115470A, in order to suppress the occurrence of erroneous determination due to deterioration of the immunochromatographic carrier, the presence or absence of deterioration of the immunochromatographic carrier is determined before the determination. At this time, in a state where the sample is developed, the change amount in brightness in a predetermined region on the immunochromatographic carrier is measured, and in a case where the change amount in brightness is larger than a predetermined standard value, it is determined that the immunochromatographic carrier has deteriorated. Accordingly, it is possible to suppress erroneous determination due to deterioration of the immunochromatographic carrier.

### SUMMARY OF THE INVENTION

In the assay apparatus, in addition to erroneous determination due to deterioration of the immunochromatographic carrier, color development due to nonspecific adsorption of a labeling substance in or in the vicinity of the assay region may result in a positive determination in spite of being originally negative. Such erroneous determination due to nonspecific adsorption of the labeling substance impairs the reliability of the immunochromatographic assay. In the assay apparatus, it is desired that an determination result with higher reliability than in the prior art is presented.

The present disclosure has been made in view of the above circumstances, and an object of the present disclosure is to provide an immunochromatographic assay apparatus capable of presenting determination results with higher reliability than in the prior art.

The immunochromatographic assay apparatus of the present disclosure includes a loading part in which a cartridge including a carrier having a spotting region on which a sample is spotted and an assay region in which a color development state changes depending on whether the sample is positive or negative, is attachably and detachably loaded,
an imaging unit that images the assay region, and
a processor configured to perform a main determination, which is a determination of whether the sample is positive or negative, based on an assay region image of the assay region imaged by the imaging unit,
in which in a case where a development direction of the sample in the carrier is a row direction and a direction intersecting the row direction is a column direction, the assay region is a line-shaped region extending along the column direction,
the assay region image is an image in which a plurality of pixels are two-dimensionally arranged in a matrix form,
the processor is configured to perform an abnormality determination for determining presence or absence of an abnormality in the assay region image, using, as a determination index, at least one of a difference between a relatively large pixel value and a relatively small pixel value in at least one column in the assay region image, a standard deviation based on pixel values in each of the columns, or a variation coefficient based on pixel values in each of the columns, or at least one of a difference between a relatively large representative value and a relatively small representative value among representative values of all rows, which is derived based on a plurality of pixels existing in different columns in the same row in the assay region image, a standard deviation based on the representative values of all the rows, or a variation coefficient based on the representative values of all the rows, and
determine a processing content related to the main determination based on the presence or absence of the abnormality.

In the immunochromatographic assay apparatus of the present disclosure, the processing content may include any one of whether or not to perform the main determination or a method of presenting a main determination result of the main determination.

In the immunochromatographic assay apparatus of the present disclosure, the processor may be configured to perform the main determination to present the main determination result in a case where the abnormality is absent, or not to perform the main determination in a case where the abnormality is present.

In the immunochromatographic assay apparatus of the present disclosure, the processor is preferably configured to present that the main determination is not performed in a case where the main determination is not performed.

In the immunochromatographic assay apparatus of the present disclosure, the processor may be configured to present that the assay region may be stained in a case where the abnormality is present.

In the immunochromatographic assay apparatus of the present disclosure, the processor may be configured to present a determination result of the main determination in a case where the abnormality is absent, and present the determination result of the main determination with reservation indicating that the assay region image has an abnormality or not present the determination result of the determination in a case where the abnormality is present and in a case where the determination result of the main determination is positive.

In the immunochromatographic assay apparatus of the present disclosure, the processor may be configured to perform the main determination, and perform the abnormality determination in a case where a determination result is positive.

In the immunochromatographic assay apparatus of the present disclosure, the processor may be configured to present a determination result of the main determination in a case where the abnormality is absent, or present the determination result of the main determination with reservation indicating that the assay region image has an abnormality or not present the determination result in a case where the abnormality is present.

In the immunochromatographic assay apparatus of the present disclosure, an imaging unit may be an image sensor that images an observation region including the assay region to output an observation image including the observation region, the observation image is an image in which a plurality of pixels are two-dimensionally arranged in a matrix form, and the processor may be configured to derive a profile in the row direction based on the representative values of the respective columns in the observation image and extract the assay region image corresponding to the assay region from the observation image based on the derived profile.

According to the immunochromatographic assay apparatus of the present disclosure, a determination result with higher reliability than in the prior art can be presented.

### BRIEF DESCRIPTION OF THE DRAWINGS

Fig. 1 is a perspective view showing appearance of an immunochromatographic assay apparatus according to the first embodiment.
Fig. 2 is a perspective view of a cartridge.
Fig. 3 is an exploded perspective view of the cartridge.
Fig. 4 is a diagram showing a positional relationship between an assay strip, a multifunctional member, a first reagent holding part, and a second reagent holding part, in the cartridge.
Fig. 5 is an explanatory diagram of an immunochromatographic method.
Fig. 6 is a partially broken side view of an assay apparatus in a state where the cartridge is loaded.
Fig. 7 is a partially broken side view of the assay apparatus in a state where the cartridge is loaded and a second reagent supply mechanism is operated.
Fig. 8 is a diagram schematically showing an observation image and an assay region image.
Fig. 9A and Fig. 9B are captured images, and Fig. 9C is a diagram showing displacement of pixel values in one pixel sequence extracted from each of the first assay region L1A of Fig. 9A and the second assay region L1B of Fig. 9B.
Fig. 10 is an explanatory diagram of data processing for deriving a determination index of abnormality determination.
Fig. 11 is an explanatory diagram of a method of extracting an assay region image.
Fig. 12 is a diagram showing a processing procedure of abnormality determination.
Fig. 13 is a diagram showing a first assay flow.
Fig. 14 is a diagram showing an assay flow performed by an immunochromatographic assay apparatus.
Fig. 15 is a diagram showing a first example of a detailed processing procedure of a main determination accompanied by abnormality determination.
Fig. 16 is a diagram showing a second example of a detailed processing procedure of a main determination accompanied by abnormality determination.
Fig. 17 is a diagram showing a third example of a detailed processing procedure of a main determination accompanied by abnormality determination.
Fig. 18 is a diagram showing a fourth example of a detailed processing procedure of a main determination accompanied by abnormality determination.
Fig. 19 is a diagram showing a fifth example of a detailed processing procedure of a main determination accompanied by abnormality determination.

### DESCRIPTION OF THE PREFERRED EMBODIMENTS

Embodiments of the immunochromatographic assay apparatus of the present disclosure will be described with reference to the drawings.

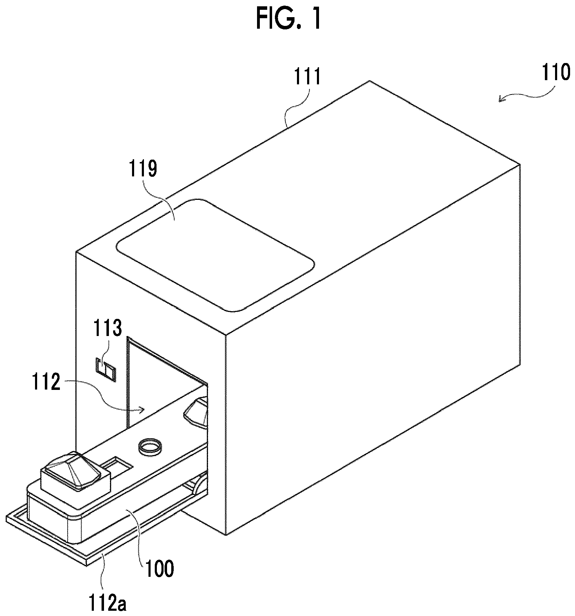

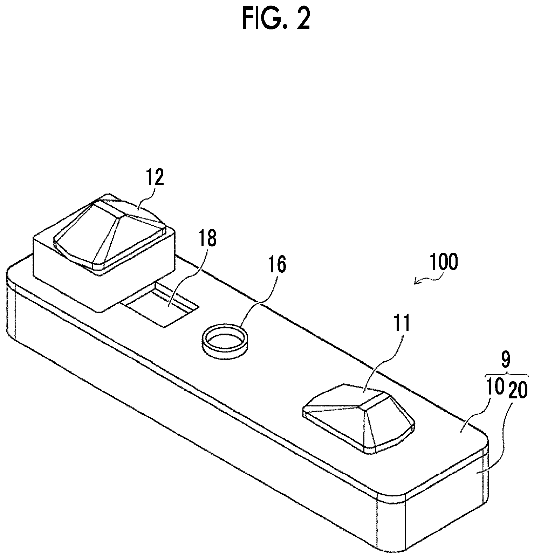

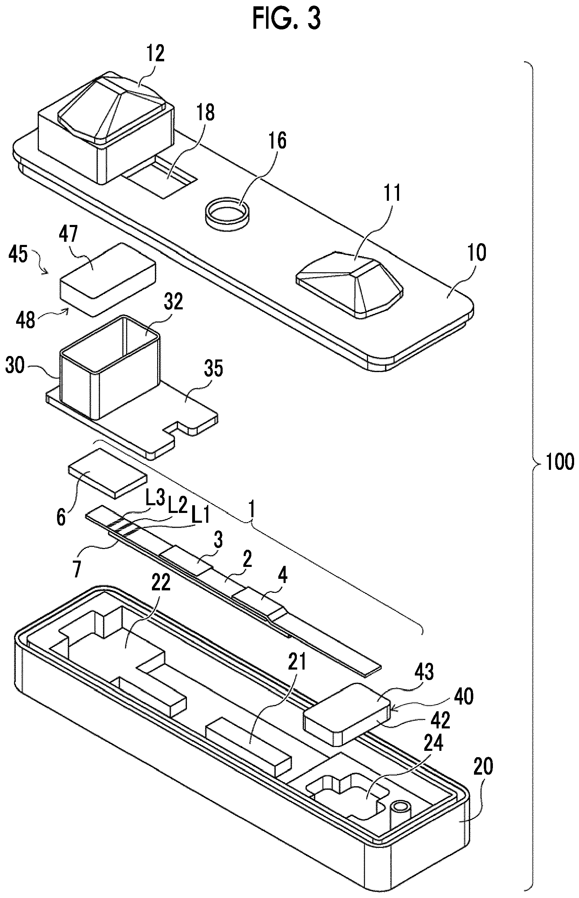

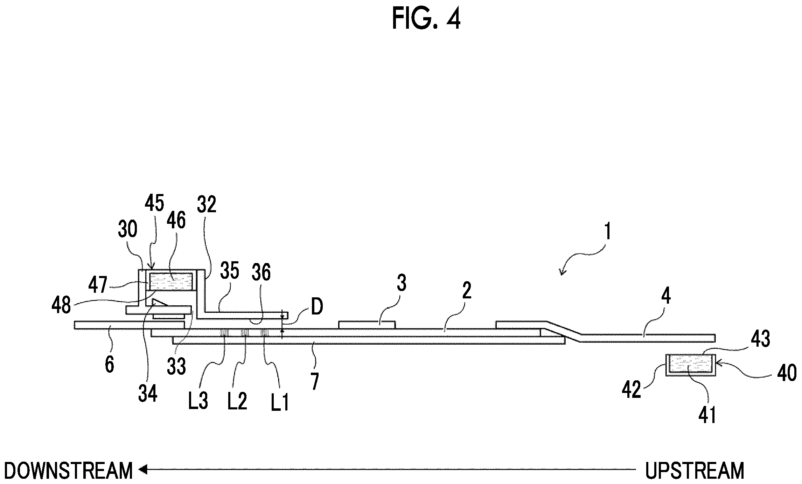

Fig. 1 is a perspective view illustrating the appearance of the immunochromatographic assay apparatus 110 (hereinafter, simply referred to as an assay apparatus 110) according to the first embodiment. Fig. 2 is an external view of a cartridge 100 mounted on the assay apparatus 110 and Fig. 3 is an exploded perspective view of the cartridge 100. Fig. 4 is a diagram illustrating the positional relationship of the main accommodated components in the cartridge 100.

The cartridge 100 is a single-use type that is used one by one for each sample of assay target. As shown in Fig. 3, an assay strip 1 including an immunochromatographic carrier 2 (hereinafter, referred to as a carrier 2) is provided in the cartridge 100. An assay region L1 is provided in the carrier 2, and the color development state changes depending on whether or not the sample contains a test substance, that is, whether the sample is positive or negative.

The sample is simply required to be a specimen that may contain a test substance, and the sample is not particularly limited. The sample is, for example, a biological specimen, particularly body fluid or excrement of an animal (particularly, a human) such as blood, serum, blood plasma, spinal fluid, tear fluid, sweat, urine, pus, nasal mucus, nasal swab, throat swab, nasal aspirate, or sputum, an organ, a tissue, a mucous membrane and skin, or swabs containing them, a liquid specimen containing animals and plants themselves or a dried body thereof. Examples of the test substance include an antigen, an antibody, a protein, and a low-molecular-weight compound.

In the assay apparatus 110 of the present example, the cartridge 100 in a state in which the sample is spotted is loaded. Then, the assay apparatus 110 detects a color development state of the assay region L1 of the loaded cartridge 100, determines whether the sample is positive or negative, and presents the assay result. Hereinafter, a determination as to whether the sample is positive or negative will be referred to as a main determination. In a case of where a plurality of samples are assayed, the cartridge 100 for each sample is loaded one by one into the assay apparatus 110.

Hereinafter, the cartridge 100 will be described on the premise that the cartridge 100 is loaded into the assay apparatus 110. However, the cartridge 100 of the present example has a configuration that a user can confirm visually whether the sample is positive or negative without using the assay apparatus 110. Such a cartridge 100 is also referred to as an immunochromatographic assay tool, an immunochromatographic assay kit, or the like.

As shown in Fig. 1, the assay apparatus 110 includes a case body 111, and the case body 111 includes a loading part 112 in which the cartridge 100 is attachably and detachably loaded. As an example, an opening for inserting the cartridge 100 into the case body 111 and an opening/closing lid 112a for opening and closing the opening are provided on the front surface of the case body 111. The opening/closing lid 112a is opened when the cartridge 100 is loaded, the cartridge 100 is inserted into the case body 111, and the opening/closing lid 112a is closed after the cartridge 100 has been loaded into the loading part 112. The assay is performed in a state where the opening/closing lid 112a is closed.

In addition, a power switch 113 is provided on the front surface of the case body 111, and a monitor 119 is provided on the upper surface of the case body 111. An assay result, an error message, and the like are displayed on the monitor 119. As an example, the monitor 119 is a touch panel monitor, and various operation screens are displayed. Through the operation screen, the user can input a start instruction of processing and an operation instruction such as selection of an assay procedure.

As shown in Fig. 2 and Fig. 3, as an example, the cartridge 100 includes a housing 9 constituted of a case member 20 and a cover member 10. The housing 9 is formed of, for example, a resin material. An opening is formed in an upper part of the case member 20, and in addition to the assay strip 1, a first reagent holding part 40, a second reagent holding part 45, and the like are accommodated therein. The cover member 10 covers the opening of the case member 20 by being attached to the opening part of the case member 20. The housing 9 has an elongated shape as a whole in accordance with the elongated shape of the assay strip 1.

In the present example, a dropping port 16, an observation window 18, a first pressed part 11, and a second pressed part 12 are provided on an upper part of the housing 9 constituted of the cover member 10. Each of these parts is integrally molded with the cover member 10 as an example. The dropping port 16 is an opening for adding dropwise a sample into the inside of the housing 9. A boss is vertically provided on the edge of the dropping port 16 toward the upper part. The observation window 18 is a window for observing the assay region L1 from the outside, and is formed of a transparent member as an example. In the present example, the size of the observation window 18 is a size such that, in addition to the assay region L1, the control region L2 and the color development region L3, which will be described later, can also be observed.

The first pressed part 11 is an operating part operated to supply the first reagent 41 (see Fig. 4) in the first reagent holding part 40 to the carrier 2. The second pressed part 12 is an operating part operated to supply the second reagent 46 in the second reagent holding part 45 to the carrier 2. As will be described later, the first reagent 41 and the second reagent 46 are amplification agents for amplifying the color development in the assay region L1 in a case where a sample 50 is positive.

In a case where a pressing force is applied from the outside as an external force to the first pressed part 11, the first pressed part 11 is deformed. As an example, the first pressed part 11 has a quadrangular pyramid shape, and in a case where a pressing force is applied from above to a region including the apex of the quadrangular pyramid, the first pressed part 11 is deformed such that the apex of the quadrangular pyramid sinks into the inside of the housing 9. In a case of where the first pressed part 11 is deformed in this manner, a pressing force is applied to the first reagent holding part 40 inside the housing 9. In the first reagent holding part 40, deformation or the like due to a pressing force applied through the first pressed part 11 occurs. Due to this deformation or the like, the first reagent 41 held by the first reagent holding part 40 is supplied to the assay strip 1.

In addition, it is preferable that the first pressed part 11 is deformed by pressing and then the deformed state is maintained. The reason is as follows. As will be described later, in the assay apparatus 110 of the present example, the cartridge 100 in a state in which the first pressed part 11 is pressed in advance by the user can be loaded. It is because, in a case where the first pressed part 11 is pressed by the user before being loaded into the assay apparatus 110, the first pressed part 11 in which the deformation is maintained even after the user releases the hand, is easier to continue the supply of the first reagent 41.

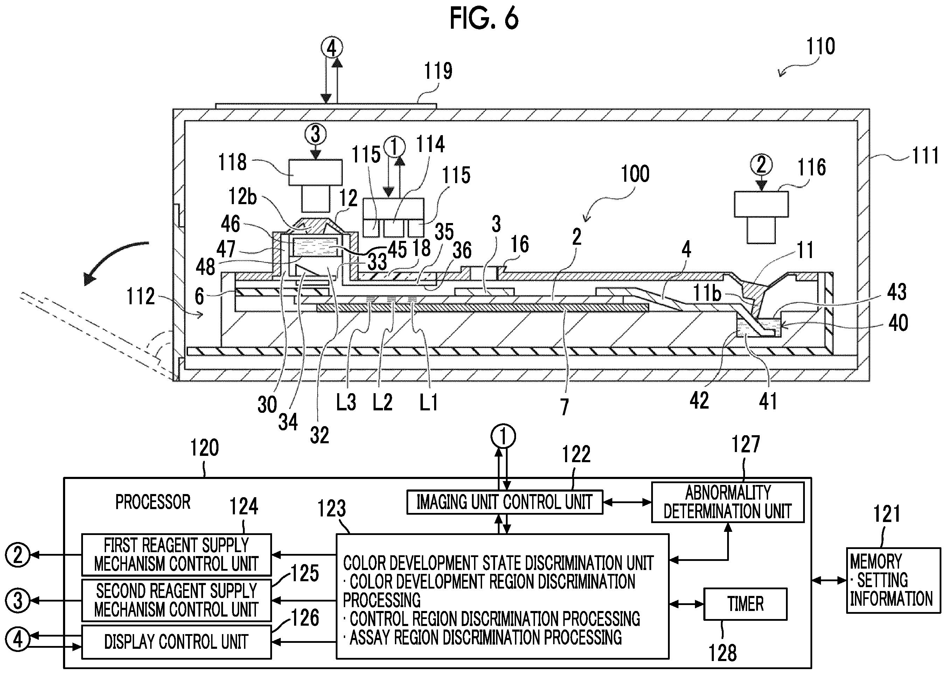

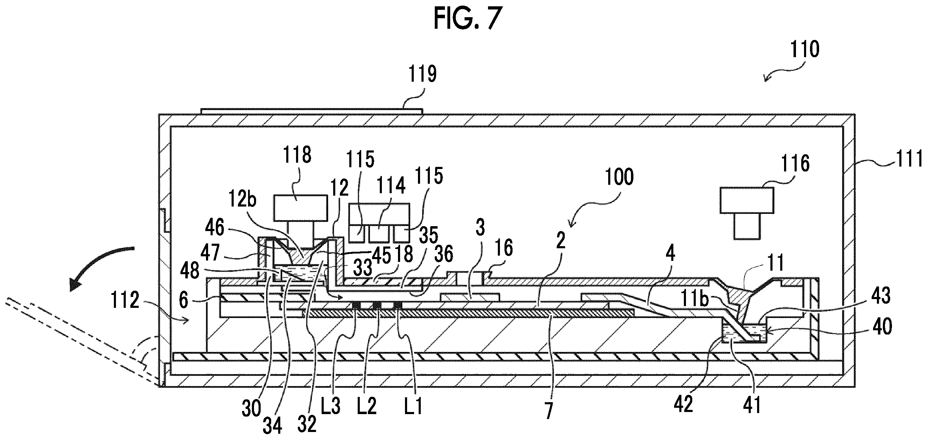

Similarly, in a case where a pressing force is applied from the outside as an external force to the second pressed part 12, the second pressed part 12 is deformed. Similarly to the first pressed part 11, the second pressed part 12 of the present example also has a quadrangular pyramid shape, and in a case where a pressing force is applied from above to a region including the apex of the quadrangular pyramid, the second pressed part 12 is deformed such that the apex of the quadrangular pyramid sinks into the inside of the housing 9. In a case of where the second pressed part 12 is deformed in this manner, a pressing force is applied to the second reagent holding part 45 inside the housing 9. In the second reagent holding part 45, deformation or the like due to a pressing force applied through the second pressed part 12 occurs. Due to this deformation or the like, the second reagent 46 held by the second reagent holding part 45 is supplied to the assay strip 1. In the second pressed part 12 of the present example, an abutting part 12b that abuts on the second reagent holding part 45 is provided (see Fig. 6 and Fig. 7).

As will be described later, in the assay apparatus 110 of the present example, a plurality of assay flows can be selected. In a case where in the assay apparatus 110, the assay flow for supplying the second reagent 46 is selected from the assay flows that can be selected in the assay apparatus 110, the second pressed part 12 is pressed by the internal mechanism of the assay apparatus 110. Therefore, the second pressed part 12 may be depressible by an internal mechanism. In a case of selecting an assay flow in which the cartridge 100 is loaded into the assay apparatus 110 after the supply of the first reagent 41 and the second reagent 46, the cartridge 100 is preferably an aspect that the second pressed part 12 is also depressible by the user.

As shown in Fig. 3 and Fig. 4, the case member 20 accommodates the assay strip 1 including the carrier 2 along the longitudinal direction. In the case member 20, the first reagent holding part 40 is disposed on one end part side (upstream side shown in Fig. 4) in the longitudinal direction. In the case member 20, in a portion where the first reagent holding part 40 is disposed, the first accommodating part 24 that is a recess-shaped in accordance with the shape of the first reagent holding part 40 is formed. One end part of the assay strip 1 is disposed above the first reagent holding part 40 in a state of being accommodated in the first accommodating part 24.

The first reagent holding part 40 holds the first reagent 41. The first reagent holding part 40 is constituted of, for example, a container 42 formed of a resin material and having an opening on one surface, and a sheet member 43 that covers the opening of the container 42 and is breakable. The container 42 is filled with the first reagent 41, and the opening of the container 42 is sealed by the sheet member 43. The first reagent holding part 40 is disposed in the first accommodating part 24 in a posture in which the sheet member 43 faces upward. The pressing force applied from the first pressed part 11 is transmitted to the sheet member 43 of the first reagent holding part 40 via the end part of the assay strip 1 to break the sheet member 43 (see Fig. 6 and Fig. 7). The sheet member 43 is broken, and thus the first reagent 41 is supplied to the assay strip 1. In the first pressed part 11 of the present example, a protruding part 11b that abuts on the sheet member 43 (see Fig. 6 and Fig. 7). The protruding part 11b has, for example, an elongated shape extending in the longitudinal direction in the width direction of the assay strip 1 and a pointed shape toward the sheet member 43, such that the sheet member 43 is easily broken.

In addition, the cartridge 100 includes a multifunctional member 30 having a function of accommodating the second reagent holding part 45. The multifunctional member 30 is disposed on the other end part side (downstream side shown in Fig. 4) of the case member 20 and above the assay strip 1. The multifunctional member 30 is a member in which the second accommodating part 32 and the flow channel forming part 35 are integrally formed. The second accommodating part 32 is a part accommodating the second reagent holding part 45. The second accommodating part 32 has a box shape having an opened upper surface. As shown in Fig. 4, on the bottom of the second accommodating part 32, a protrusion 34 for breaking a sheet member 48, which will be described later, of the second reagent holding part 45, and an opening 33 that allows to flow the second reagent 46 flowed out from the second reagent holding part 45, toward the carrier 2.

The flow channel forming part 35 is provided to be connected to the upstream side from the second accommodating part 32. The flow channel forming part 35 has a flat plate shape, is disposed at a position facing the assay region L1 or the like in the longitudinal direction of the carrier 2, and is disposed with an interval from the carrier 2. Then, between the flow channel forming part 35 and the carrier 2, a flow channel for flowing the second reagent 46 flowed out from the second accommodating part 32 toward the assay region L1 or the like is formed. In this way, the flow channel forming part 35 is disposed between the observation window 18 and the assay region L1 or the like of the carrier 2. Therefore, the flow channel forming part 35 is formed of a transparent member and thus the assay region L1 and the like can be observed through the observation window 18.

The second reagent holding part 45 holds the second reagent 46. The second reagent holding part 45 is constituted of, for example, a container 47 formed of a resin material and having an opening on one surface, and a sheet member 48 that covers the opening of the container 47 and is breakable. The container 47 is filled with the second reagent 46, and the opening of the container 47 is sealed by the sheet member 48. The second reagent holding part 45 is disposed in the second accommodating part 32 in a posture in which the sheet member 48 faces downward. Accordingly, the sheet member 48 faces the protrusion 34 in the second accommodating part 32.

The pressing force applied from the second pressed part 12 to the second reagent holding part 45 acts in a direction of pushing down the second reagent holding part 45 downwardly, whereby the sheet member 48 is pressed against the protrusion 34. The sheet member 48 is pressed against the protrusion 34, whereby the sheet member 48 is broken (see Fig. 6 and Fig. 7). The sheet member 48 is broken, and thus the second reagent 46 is supplied to the carrier 2 through the flow channel formed by the opening 33 at the bottom of the second accommodating part 32 and the flow channel forming part 35.

As shown in Fig. 4, a gap (a clearance) D corresponding to the flow channel for the second reagent 46 is formed between a back surface 36 of the flow channel forming part 35 of the multifunctional member 30 and the carrier 2 of the assay strip 1. The gap D is, for example, in the range of 0.01 mm to 1 mm. The second reagent 46 flows out from the opening 33 at the bottom of the second accommodating part 32 toward the carrier 2, and the second reagent 46 that has flowed out flows through the flow channel formed by the gap D and reaches at least above the assay region L1. The second reagent 46 that has reached the assay region L1 infiltrates the assay region L1 from the flow channel.

An absorption pad 6, which will be described later, is disposed at an end part on the downstream side of the assay strip 1. In the case member 20, a support part 22 that supports an end part of the assay strip 1 including the absorption pad 6 is formed at a position facing the absorption pad 6. A second accommodating part 32 of the multifunctional member 30 is disposed above the absorption pad 6. The support part 22 also supports the multifunctional member 30 via the absorption pad 6. In addition, in the case member 20, a support part 21 that supports a central part of the assay strip 1 is formed.

The assay strip 1 includes a carrier 2, a liquid feeding pad 4, and an absorption pad 6. Then, the carrier 2 is fixedly supported on a back pressure-sensitive adhesive sheet 7.

The carrier 2 is a porous insoluble carrier for developing a sample, and includes an assay region L1, a control region L2, and a color development region L3. In addition, the carrier 2 includes a label holding pad 3. The label holding pad 3 constitutes a spotting region on which the sample is spotted. The color development region L3 is disposed on the downstream side of the assay region L1 in a case where the direction toward the assay region L1 with respect to the spotting region is the downstream side of the carrier 2. In the present example, the assay region L1, the control region L2, and the color development region L3 are line-shaped regions extending in a direction perpendicular to the development direction of the sample in the carrier 2.

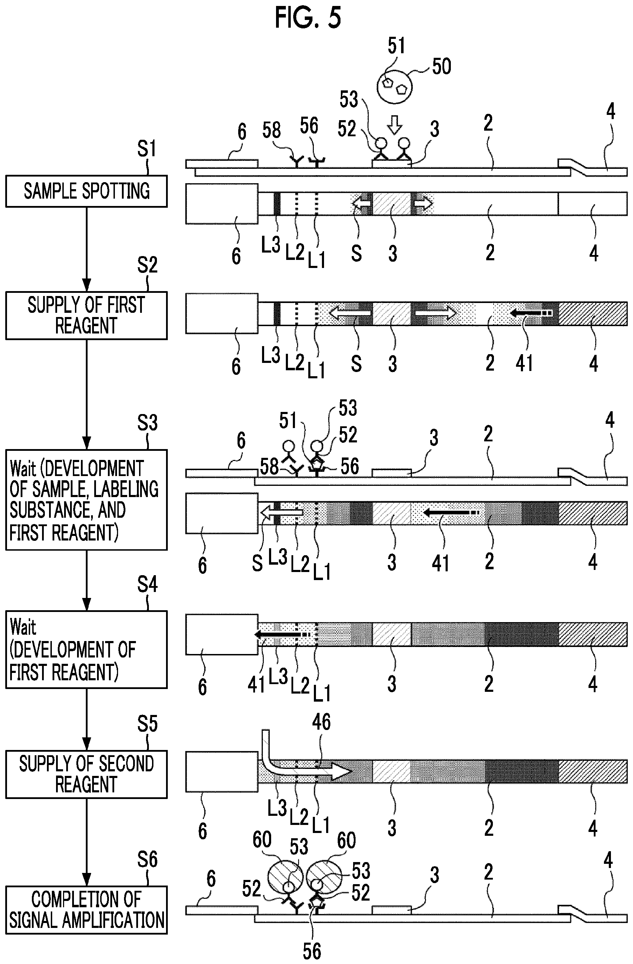

It shows a state in which the assay region L1, the control region L2, and the color development region L3 are expressed as lines, but these are not always expressed. Details will be described later, but before developing the sample 50 (see Fig. 5), the first reagent 41 (see Fig. 4), and the second reagent 46 (see Fig. 4), the colors of the assay region L1 and the control region L2 are substantially the same as the color of the carrier 2 (for example, white), and thus the assay region L1 and the control region L2 cannot be clearly visually recognized at this stage. The assay region L1 is expressed as a line by increasing the color optical density in a case where the sample 50 is developed and the developed sample 50 is positive. As a result, the assay region L1 becomes visible. Since the color development of the assay region L1 is amplified by silver amplification, which will be described later, the assay region L1 develops a black color.

The control region L2 is also expressed as a line by increasing the color optical density in a case where the sample 50 is developed. As a result, the control region L2 becomes visible. Since the color development of the control region L2 is also subjected to silver amplification, the control region L2 also develops a black color.

On the other hand, only the color development region L3 is expressed and visible as a blackish dark green color (hereinafter, referred to as a dark green color) line even in a stage before the first reagent 41 is developed. However, the color development region L3 is expressed as an orange line by changing a dark green color to an orange color in a case where the first reagent 41 is developed.

As the carrier 2, for example, a porous material such as a nitrocellulose membrane can be used. In addition, the back pressure-sensitive adhesive sheet 7 on which the carrier 2 is fixed is a sheet-shaped substrate having a pressure-sensitive adhesive surface to which the carrier 2 is attached.

As shown in Fig. 5, a labeling substance 53 is fixed to the label holding pad 3. The labeling substance 53 is modified with the first binding substance 52 that specifically binds to the test substance 51 contained in the sample 50. The label holding pad 3 is fixed on the carrier 2 at a position facing the dropping port 16 of the cover member 10. Therefore, the sample 50 is added dropwise onto the label holding pad 3 from the dropping port 16. Therefore, the label holding pad 3 corresponds to a spotting region on which the sample 50 is spotted.

The label holding pad 3 is fixed at a substantially center position in the longitudinal direction of the carrier 2. As the labeling substance 53, it is possible to use, for example, a gold colloidal particle having a diameter of 50 nm (EM. GC50, manufactured by BBI Solutions). The labeling substance 53 is not limited to the gold colloid, and a metal sulfide that can be used in a general chromatographic method, a coloring particle that are used in an immunoagglutination reaction, or the like can be used, where a metal colloid is particularly preferable. Examples of the metal colloid include a gold colloid, a silver colloid, a platinum colloid, an iron colloid, an aluminum hydroxide colloid, and a composite colloid thereof. In particular, at an appropriate particle diameter, a gold colloid is preferable since it exhibits a red color, a silver colloid is preferable since it exhibits a yellow color, the gold colloid is most preferable among them.

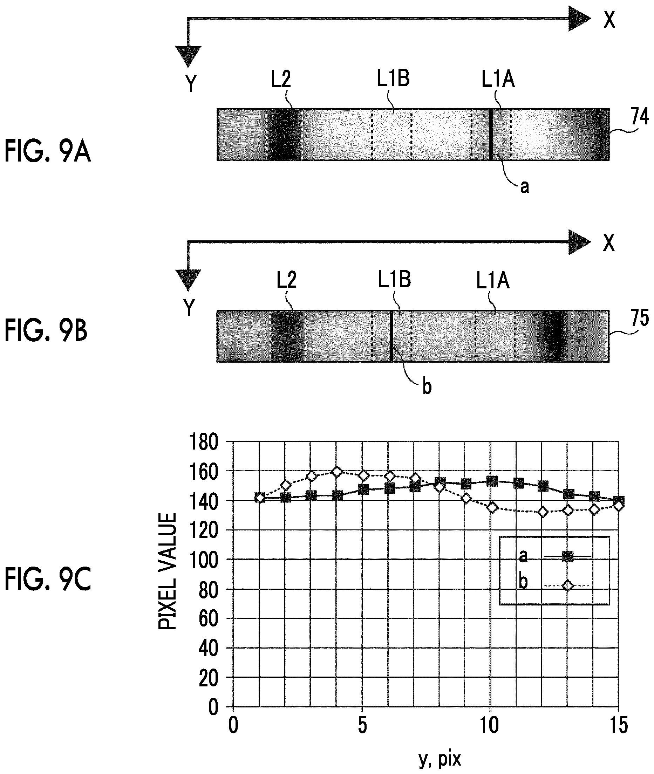

As shown in Fig. 5, the assay region L1 includes a second binding substance 56 that specifically binds to the test substance 51 and captures the test substance 51. In the assay region L1, in a case where the test substance 51 is captured by binding the second binding substance 56 to the test substance 51, the first binding substance 52 bonded to the test substance 51 and the labeling substance 53 are captured. In a case where the test substance 51 is included in the sample 50, the test substance 51 and the labeling substance 53 are captured in the assay region L1, and thus the color optical density in the assay region L1 is increased to be not less than a preset reference. The assay region L1 is a region for confirming the presence or absence of the test substance 51 by a labeling signal from the labeling substance 53 captured via the test substance 51. The test substances 51 detected by one cartridge 100 is not limited to one kind, and a plurality of assay regions L1 may be provided to simultaneously detect a plurality of different test substances 51. In a case where, for example, a first assay region L1A and a second assay region L1B are provided as the plurality of assay regions L1 (see Fig. 9), the first assay region L1A includes a second binding substance 56 that specifically binds to the first test substance 51, and the second assay region L1B includes a second binding substance 56 that specifically binds a second test substance 51 different from the first test substance 51.

The control region L2 includes a third binding substance 58 that specifically binds to the first binding substance 52, and captures the labeling substance 53 via the first binding substance 52. In a case where the sample 50 is spotted on the label holding pad 3, the labeling substance 53 that is not bound to the test substance 51 among the labeling substances 53 modified with the first binding substance 52 is also developed in the carrier 2 toward the assay region L1 together with the sample 50. The labeling substance 53 that is not bound to the test substance 51 passes through the assay region L1 without being captured by the assay region L1. The labeling substance 53 that has passed through the assay region L1 is captured in the control region L2 via the first binding substance 52 by binding the first binding substance 52 to the third binding substance 58. The labeling substance 53 is captured in the control region L2, and thus the color optical density in the control region L2 is increased to be not less than a preset reference. The control region L2 is a region for confirming the completion of the development of the sample 50 by the labeling signal from the labeling substance 53 captured via the first binding substance 52. Therefore, the control region L2 may be referred to as a confirmation region.

The first binding substance 52 that modifies the labeling substance 53 and specifically binds to the test substance 51 is a substance that specifically binds to the test substance 51, for example, in a case where the test substance 51 is an antigen, an antibody against the antigen, in the case where the test substance 51 is an antibody, an antigen against the antibody, in the case where the test substance 51 is a protein or a low-molecular-weight compound, an aptamer against the protein or the low-molecular-weight compound, or the like.

The second binding substance 56 that is fixed in the assay region L1 and specifically binds to the test substance 51 is a substance that specifically binds to the test substance 51, for example, in a case where the test substance 51 is an antigen, an antibody against the antigen, in the case where the test substance 51 is an antibody, an antigen against the antibody, in the case where the test substance 51 is a protein or a low-molecular-weight compound, an aptamer against the protein or the low-molecular-weight compound, or the like. The first binding substance 52 and the second binding substance 56 may be the same as or different from each other.

The third binding substance 58 that specifically binds to the first binding substance 52 may be the test substance 51 itself or may be a compound having a site recognized by the first binding substance 52. Examples thereof include a compound obtained by binding a derivative of the test substance 51 to a protein, and the like.

For example, in a case where the test substance 51 is an influenza A virus or a biomarker thereof, an anti-influenza A monoclonal antibody (Anti-Influenza A SPTN-5 7307, Medix Biochemica) can be used as the first binding substance 52 and the second binding substance 56, and an anti-mouse IgG antibody (anti-mouse IgG (H+L), rabbit F(ab')2, product number 566-70621, manufactured by FUJIFILM Wako Pure Chemical Corporation) can be used as the third binding substance 58.

The color development region L3 contains a substance whose color development state changes in response to the first reagent 41. The color development region L3 indicates that the first reagent 41 has been developed to that region by reacting with the first reagent 41 to develop a color or change a color. For example, in a case where a mixed aqueous solution of an iron nitrate aqueous solution and citric acid (manufactured by Fujifilm Wako Pure Chemical Corporation, 038-06925) is used as the first reagent 41, an aspect in which the color development region L3 is constituted of a color reagent immobilization line on which Bromocresol Green (manufactured by FUJIFILM Wako Pure Chemical Corporation) has been immobilized in a line shape is preferable. This aspect is the aspect of the color development region L3 of the present example. As described above, the color development region L3 of the present example is dark green color before reacting with the first reagent 41, and the dark green color is changed to an orange color in a case where the first reagent 41 reaches the color development region L3. The color development region L3 is sometimes referred to as an amplification index region because the timing of supplying the second reagent 46 after the first reagent 41 is developed is indicated by changing the color development state.

The liquid feeding pad 4 is disposed in contact with one end of the carrier 2 and the first reagent 41 is fed to the carrier 2 from the upstream side of the spotting region (constituted of the label holding pad 3). In the liquid feeding pad 4, in a case where the first pressed part 11 is pressed, one end of the liquid feeding pad 4 is immersed in the first reagent holding part 40. The liquid feeding pad 4 is formed of a porous material and absorbs the first reagent 41, and the absorbed first reagent 41 is fed to the carrier 2 by a capillary action.

The absorption pad 6 is disposed in contact with the other end of the carrier 2 and absorbs the sample 50, the first reagent 41, and the second reagent 46, which are developed on the carrier 2. The absorption pad 6 is also formed of a porous material.

In the present embodiment, the first reagent 41 and the second reagent 46 are amplification agents that amplify the color development in the assay region L1 and the control region L2 by reacting with each other. In a case where a metal-based labeling substance such as a gold colloid is used as the labeling substance 53 as in the present example, for example, silver amplification is used as a method of amplifying the labeling signal of the labeling substance 53. The first reagent 41 and the second reagent 46 are, as an example, amplification agents used for silver amplification, and the reaction between the first reagent 41 and the second reagent 46 using the labeling substance 53 as a catalyst is an amplification reaction. By the amplification reaction, silver particles 60 (see Fig. 5) having a particle diameter relatively larger than that of the labeling substance 53 are generated.

More specifically, in the present example, the first reagent 41 is a reducing agent that reduces silver ions, and the second reagent 46 is a silver ion. In a case where the first reagent 41, which is a reducing agent, and the second reagent 46, which is a silver ion, are brought into contact with the labeling substance 53, silver particles 60 (see Fig. 5) are generated, and the generated silver particles 60 deposits on the labeling substance 53 using the labeling substance 53 as a nucleus. By depositing the silver particles on the labeling substance 53, silver particles 60 having a particle diameter larger than that of the labeling substance 53 (see Fig. 5) are generated. Accordingly, the labeling signal issued by the labeling substance 53 is amplified, and as a result, the color development of the labeling substance 53 is amplified in the assay region L1 and the control region L2.

### (First Reagent)

As the reducing agent as the first reagent 41, any inorganic or organic material or a mixture thereof can be used as long as the silver ion used as the second reagent 46 can be reduced to silver. Preferred examples of the inorganic reducing agent include a reducing metal salt and a reducing metal complex salt, of which the atomic valence is capable of being changed with a metal ion such as Fe²⁺, V²⁺, or Ti³⁺. In a case where an inorganic reducing agent is used, it is necessary to remove or detoxify oxidized ions by complexing or reducing the oxidized ions. For example, in a system in which Fe²⁺ is used as the reducing agent, a complex of Fe³⁺, which is an oxide, is formed using citric acid or ethylenediaminetetraacetic acid (EDTA), which enables the detoxification of the oxidized ions. In the present system, such an inorganic reducing agent is preferably used, and it is more preferable that a metal salt of Fe²⁺ is preferably used.

It is also possible to use a developing agent used in a light-sensitive silver halide photographic material of a wet-type (for example, methyl gallate, hydroquinone, substituted hydroquinone, 3-pyrazolidones, p-aminophenols, p-phenylenediamines, hindered phenols, amidoximes, azines, catechols, pyrogallols, ascorbic acid (or a derivative thereof), and leuco dyes), and other materials obvious to those who are skilled in the related art in the present field, for example, a material described in US6020117A.

As the reducing agent, an ascorbic acid reducing agent is also preferable. The useful ascorbic acid reducing agent includes ascorbic acid, an analogue thereof, an isomer thereof, and a derivative thereof. Preferred examples thereof include D- or L-ascorbic acid and a sugar derivative thereof (for example, γ-lactoascorbic acid, glucoascorbic acid, fucoascorbic acid, glucoheptoascorbic acid, or maltoascorbic acid), a sodium salt of ascorbic acid, a potassium salt of ascorbic acid, isoascorbic acid (or L-erythroascorbic acid), a salt thereof (for example, an alkali metal salt, an ammonium salt, or a salt known in the related technical field), ascorbic acid of the enediol type, ascorbic acid of the enaminol type, ascorbic acid of the thioenol type. Particularly, D-, L-, or D,L-ascorbic acid (and an alkali metal salt thereof) or isoascorbic acid (or an alkali metal salt thereof) is preferable, and a sodium salt is a preferred salt. A mixture of these reducing agents can be used as necessary.

### (Second reagent)

The solution containing silver ions, which is used as the second reagent 46, is preferably a solution obtained by dissolving a silver ion-containing compound in a solvent. As the silver ion-containing compound, an organic silver salt, an inorganic silver salt, or a silver complex can be used. An inorganic silver salt or a silver complex is preferable. As the inorganic silver salt, it is possible to use a silver ion-containing compound having a high solubility in solvents such as water, and examples thereof include silver nitrate, silver acetate, silver lactate, silver butyrate, and silver thiosulfate. Silver nitrate is particularly preferable. The silver complex is preferably a silver complex in which silver is coordinated with a ligand having a water-soluble group such as a hydroxyl group or a sulfone group, and examples thereof include silver hydroxythioether.

### <Immunochromatographic method>

An immunochromatographic method will be described with reference to Fig. 5. Here, a case where the sample 50 includes the test substance 51, that is, on the premise that the sample 50 is positive will be described.

First, the sample 50 is spotted on the label holding pad 3 which is the spotting region (Step S1). The test substance 51 in the sample 50, which is spotted on the label holding pad 3, specifically binds to the first binding substance 52 that modifies the labeling substance 53 contained in the label holding pad 3. The sample 50 is developed on the downstream side from the label holding pad 3 in the carrier 2 by the capillary action in the carrier 2. Apart of the sample 50 is also developed on the upstream side. The arrow S indicates a state in which the sample 50 is developed.

Next, the first reagent 41 is supplied (Step S2). The first reagent 41 is supplied from the liquid feeding pad 4. The first reagent 41 is supplied to the carrier 2 via the liquid feeding pad 4 and is developed on the downstream side.

After that, the process waits until the first reagent 41 is developed on the downstream side (Step S3 and Step S4). "Wait" shown in Fig. 5 means an action of waiting. The first reagent 41 is gradually developed to the downstream side, and the sample 50 to be developed from the label holding pad 3 and the labeling substance 53 modified with the first binding substance 52 are developed to the downstream side to be pushed by the first reagent 41 (Step S3).

The test substance 51 in the sample 50 that has been developed to the downstream side and has reached the assay region L1 is captured by the second binding substance 56 of the assay region L1. That is, the labeling substance 53 is captured in the assay region L1 via the test substance 51 and the first binding substance 52. On the other hand, the labeling substance 53 that is not bound to the test substance 51 passes through the assay region L1 without being captured and is captured by the third binding substance 58 of the control region L2.

In a case where the development of the first reagent 41 proceeds and the first reagent 41 reaches the color development region L3 (Step S4), the color development region L3 reacts with the first reagent 41 to change the color development state. In the present example, the color development region L3 is dark green color before reacting with the first reagent 41, and the dark green color is changed to an orange color by reacting with the first reagent 41.

After the first reagent 41 is sufficiently developed, the second reagent 46 is supplied to the carrier 2 (Step S5). The second reagent 46 is supplied to the carrier 2 from the downstream side of the color development region L3 and is developed on the upstream side. Here, the first reagent 41 is a first amplifying liquid containing a reducing agent that reduces silver ions, and the second reagent 46 is a second amplifying liquid containing silver ions. By reacting the first amplifying liquid with the second amplifying liquid, the silver particles 60 are generated using the gold colloidal particles that are the labeling substance 53 as a catalyst. Accordingly, the labeling signal is amplified (Step S6).

Fig. 6 and Fig. 7 are partially broken side views of the assay apparatus 110 in a state where the cartridge 100 is loaded. Hereinafter, a configuration and a function of the assay apparatus 110 will be described with reference to Fig. 6 and Fig. 7.

In the assay apparatus 110 of the present example, for example, the following three assay flows of a first assay flow, a second assay flow, and a third assay flow can be selected. In any of the first to third assay flows, it is necessary that the sample 50 is spotted on the carrier 2 of the cartridge 100 before loading.

The first assay flow is a flow in which an assay is performed on the cartridge 100 in a state where the spotting of the sample 50 and the supply of the first reagent 41 are started before loading. In the case of the first assay flow, after the cartridge is loaded into the assay apparatus 110, only the supply of the second reagent 46 to the carrier 2, among the first reagent 41 and the second reagent 46, is performed by the assay apparatus 110.

The second assay flow is a flow in which an assay is performed on the cartridge 100 in which only the spotting of the sample 50 is performed before loading. In the case of the second assay flow, after the cartridge is loaded into the assay apparatus 110, the supply of both the first reagent 41 and the second reagent 46 to the carrier 2 is performed by the assay apparatus 110.

The third assay flow is a flow in which an assay is performed on the cartridge 100 in a state where the spotting of the sample 50, the supply of the first reagent 41, and the supply of the second reagent 46 are started before loading. In the case of the third assay flow, after the cartridge is loaded into the assay apparatus 110, the supply of the first reagent 41 and the second reagent 46 is not performed in the assay apparatus 110.

Hereinafter, a configuration of the assay apparatus 110 will be described, and then a first assay flow will be described.

### (Configuration of assay apparatus 110)

As shown in Fig. 6 and Fig. 7, the assay apparatus 110 includes a first reagent supply mechanism 116 and a second reagent supply mechanism 118 as internal mechanisms. The first reagent supply mechanism 116 is a mechanism for starting the supply of the first reagent 41 from the first reagent holding part 40 to the carrier 2. As the first reagent supply mechanism 116, for example, an actuator such as a solenoid provided with an electromagnet and a plunger movable with respect to the electromagnet is used. For example, as the plunger moves, the plunger abuts on the first pressed part 11 and presses the first pressed part 11. The first reagent supply mechanism 116 is disposed at a position facing the first pressed part 11 of the loaded cartridge 100.

The first reagent supply mechanism 116 is a pressing mechanism that applies a pressing force to the first pressed part 11 from the outside by pressing the first pressed part 11 of the cartridge 100. In a case where a pressing force is applied to the first pressed part 11 by the first reagent supply mechanism 116, the first reagent 41 is supplied from the first reagent holding part 40 to the carrier 2 by the above-described action. In the first assay flow and the third assay flow, the first reagent supply mechanism 116 is not used and only in the second assay flow, the first reagent supply mechanism 116 is used.

The second reagent supply mechanism 118 is a mechanism for starting the supply of the second reagent 46 from the second reagent holding part 45 to the carrier 2. Also as the second reagent supply mechanism 118, an actuator such as a solenoid is used similarity to the first reagent supply mechanism 116. The second reagent supply mechanism 118 is disposed at a position facing the second pressed part 12 of the loaded cartridge 100. The second reagent supply mechanism 118 is a pressing mechanism that applies a pressing force to the second pressed part 12 from the outside by pressing the second pressed part 12 of the cartridge 100. In a case where a pressing force is applied to the second pressed part 12 by the second reagent supply mechanism 118, the second reagent 46 is supplied from the second reagent holding part 45 to the carrier 2 by the above-described action. In the third assay flow, the second reagent supply mechanism 118 is not used and only in the first assay flow and the second assay flow, the second reagent supply mechanism 118 is used.

In addition to the loading part 112, the first reagent supply mechanism 116, and the second reagent supply mechanism 118, the assay apparatus 110 further includes an imaging unit 114, a processor 120, and a memory 121 in the case body 111. In Fig. 6, the processor 120 and the memory 121 are illustrated outside the case body 111 of the assay apparatus 110, but this is a schematic diagram and the processor 120 and the memory 121 are actually disposed inside the case body 111.

The imaging unit 114 images an observation region including at least the assay region L1 and the control region L2, and outputs the observation image of the observation region to the processor 120. It is more desirable that the observation region includes the color development region L3.

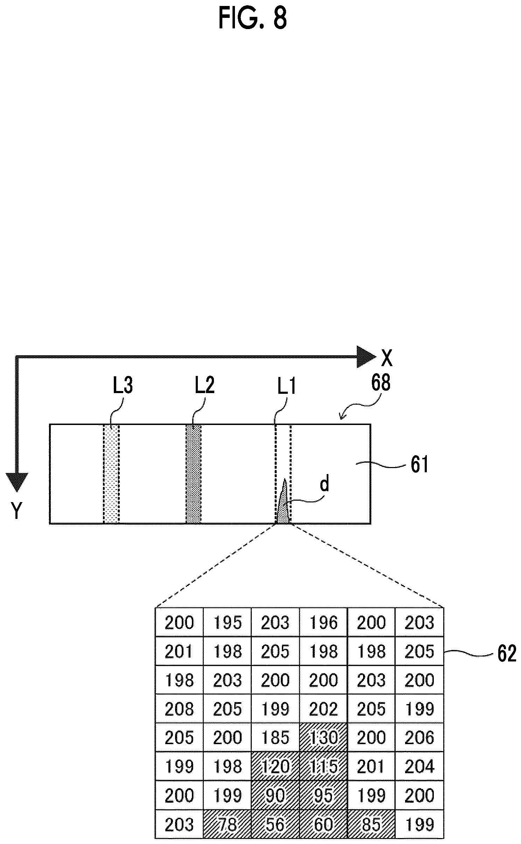

The imaging unit 114 is, for example, an image sensor such as a complementary metal oxide semiconductor (CMOS) image sensor and a charge coupled device (CCD) image sensor. The imaging unit 114 is disposed, for example, at a position facing the observation window 18. The imaging unit 114 images a preset range including the observation window 18 as the center and the periphery thereof. The captured image imaged by the imaging unit 114 includes an observation region 68 (see Fig. 8) exposed from the observation window 18, such as an assay region L1, a control region L2, and a color development region L3. Then, the captured image is output from the imaging unit 114 to the processor 120.

In addition, as an example, a light source 115 such as a light emitting diode that illuminates the assay region L1, the control region L2, and the color development region L3 during the imaging is provided on both sides of the imaging unit 114.

The processor 120 integrally controls each part of the assay apparatus 110. An example of the processor 120 is a Central Processing Unit (CPU) that performs various types of control by executing a program. The CPU functions as a control unit having an imaging unit control unit 122, a color development state discrimination unit 123, a first reagent supply mechanism control unit 124, a second reagent supply mechanism control unit 125, a display control unit 126, the abnormality determination unit 127, and a timer 128 by executing a program. The memory 121 is an example of a memory connected to or built in the CPU as the processor 120. For example, a control program is stored in the memory 121. The processor 120 is realized by the CPU executing a control program.

The imaging unit control unit 122 controls the imaging timing by the imaging unit 114.

The first reagent supply mechanism control unit 124 operates the first reagent supply mechanism 116 to control the first pressed part 11 to be pressed.

The second reagent supply mechanism control unit 125 operates the second reagent supply mechanism 118 based on the change in the color development state of the color development region L3 to control the second pressed part 12 to be pressed.

The color development state discrimination unit 123 executes the color development region discrimination processing, the control region discrimination processing, and the assay region discrimination processing based on the captured image acquired via the imaging unit 114. As described above, the imaging unit 114 outputs the captured image of the observation region 68 including the assay region L1, the control region L2, and the color development region L3. The color development state discrimination unit 123 executes each of the above-described discrimination processing based on the captured image.

The color development region discrimination processing is a processing of discriminating, based on the captured image, a change in the color development state of the color development region L3, as an example, whether or not the color has changed from a dark green color, which is the color before the reaction with the first reagent 41, to an orange color. "Presence" of the change in the color development state means that the first reagent 41 is developed to the color development region L3.

The "change in color development state" includes any of an aspect in which a first color different from the color of the carrier 2 changes to another second color (that is, a color change), an aspect in which the color of the carrier 2 changes to another color by developing a color different from that of the carrier 2 (that is, color development), and an aspect in which the density of the color changes (that is, a change in density).

The processor 120 operates the second reagent supply mechanism 118 via the second reagent supply mechanism control unit 125 in a case where the color development state discrimination unit 123 discriminates that the color development state in the color development region L3 has changed.

The control region discrimination processing is a processing of discriminating presence or absence of a change in the color development state of the control region L2 based on the captured image. In the present example, since a line is expressed in the control region L2 by the labeling substance 53 being captured in the control region L2, or the silver amplification after capturing the labeling substance 53, presence or absence of the expression of the line in the control region L2 is discriminated. In a case where it is discriminated that the color development state of the control region L2 is changed, that is, the expression in the control region L2 is present, the color development state discrimination unit 123 executes the assay region discrimination processing of the next step.

The assay region discrimination processing is a processing of discriminating presence or absence of a change in the color development state of the assay region L1 based on the captured image. In the present example, since a line is expressed in the assay region L1 by the labeling substance 53 being captured in the assay region L1, or the silver amplification after capturing the labeling substance 53, presence or absence of the expression of the line in the assay region L1 is determined as the presence or absence of the change in the color development state. A case where the line is expressed in the assay region L1 means that the sample 50 is positive, and a case where the line is not expressed means that the sample 50 is negative. The discrimination of the presence or absence of a change in the color development state of the assay region L1 in the assay region discrimination processing corresponds to the main determination of whether the sample 50 is positive or negative.

The abnormality determination unit 127 performs abnormality determination for determining presence or absence of abnormality in the assay region image. Specifically, the abnormality determination unit 127 performs the abnormality determination using, as determination index, at least one of difference in pixel value between a relatively large pixel value and a relatively small pixel value in at least one column in the assay region image, a standard deviation of the pixel values of the pixels included in the at least one column in the assay region image, or a variation coefficient based on the pixel value of each column. The abnormality determination by the abnormality determination unit 127 may be performed before or after the main determination. The method and the process of determining abnormality will be described later.

The processor 120 determines the processing content related to the main determination based on the result of the abnormality determination, that is, the presence or absence of the abnormality. The processing content related to the main determination includes, for example, at least one of whether or not to perform the main determination and presentation directions the main determination result of the main determination. Specifically, for example, the processor 120 determines that, in a case where it is determined that abnormality is present, the processing ends without performing the main determination, and only in a case where it is determined that the abnormality is absent, the main determination is performed. In addition, for example, in a case where it is determined that the abnormality is present, the processor 120 presents the result of main determination as the assay result with reservation indicating that the abnormality is present, or presents the assay result that the determination is defective without presenting the result of main determination. In addition, for example, in a case where it is determined that the abnormality is absent, the processor 120 determines to present the determination result of the main determination as the assay result as it is. In a case where it is determined that abnormality is present, the processor 120 displays a assay result of "positive with reservation" or "defective determination" on the monitor 119 via the display control unit 126. In addition, in a case where it is determined that abnormality is absent, the processor 120 displays a assay result of "positive" or the like on the monitor 119 via the display control unit 126.

In addition to the control program, the memory 121 stores setting information that is preset in order for the processor 120 to perform various types of control. As the setting information, information necessary for the color development state discrimination unit 123 to discriminate a change in the color development state is stored. Examples of the setting information include a first setting time t1 that is preset, a second setting time t2 that is preset, and a number of times K that is preset, which will be described later. The first setting time t1 is a waiting time until the processor 120 discriminates the presence or absence of a change in the color development state of the color development region L3 again in a case where the processor 120 has been determined that a change in the color development state of the color development region L3 is absent. The second setting time t2 is an allowable time in a case where the processor 120 repeatedly discriminates presence or absence of the change in the color development state again in a case where the processor 120 determines that the change in the color development state of the color development region L3 is absent. In addition, as the setting information, determination reference in a case where the processor 120 performs the abnormality determination is stored.

Here, a method of determining an abnormality by the abnormality determination unit 127 will be described with reference to Fig. 8 to Fig. 10.

First, abnormality that may occur in the assay region image will be described. Fig. 8 is a diagram schematically showing the observation image 61 acquired by cutting out the observation region 68 from the captured image output by the imaging unit 114, and the assay region image 62.

The observation image 61 is an image in which a plurality of pixels are two-dimensionally arranged in a matrix form, and the assay region image 62 extracted from the observation image 61 is also an image in which a plurality of pixels are two-dimensionally arranged in a matrix form. In a case where the length direction of the carrier 2 is defined as an X direction and the length direction of the line of the assay region L1, which intersects the X direction, is defined as a Y direction, the row direction indicates the X direction and the column direction indicates the Y direction. As shown as an example in Fig. 8, the assay region image 62 is composed of pixels arranged in a matrix form of 8 rows and 6 columns. The processor 120 converts the signal value for each pixel of the captured image output by the imaging unit 114 into a digital pixel value of 0 to 255. In Fig. 8, each pixel of the assay region image 62 shows a pixel value thereof. The pixel value of the assay region image 62 corresponds to, for example, the brightness in proportion to the amount of received light incident on each pixel. Therefore, in the assay region image 62, the higher the brightness is, the larger the pixel value is, and the lower the brightness is, the smaller the pixel value is. In a case where such pixels of the assay region image 62 are represented by the level of an optical density (hereinafter, simply referred to as a density), the smaller the pixel value is, the higher the density is, and the larger the pixel value is, the lower the density is. Here, the pixel value and the density value can be converted by a preset conversion expression.

In the observation image 61 of the example shown in Fig. 8, the lower region in the assay region L1 includes a portion d having a partial high density (hereinafter, referred to as a high-density portion d). In a case where the sample 50 is positive, since the color optical density increases on average in substantially the entire region of the assay region L1, the same line as that of the control region L2 of the observation image 61 is expressed. On the other hand, it is presumed that the high-density portion d partially generated in the assay region L1 is a portion where the color optical density is increased due to the nonspecific adsorption of the labeling substance. Such a high-density portion d is an unnecessary stain that induces an erroneous determination in the main determination. In some cases, the high-density portion d shows a density that is abnormally higher than the color optical density in a case where the sample 50 is positive. Such a high-density portion d has a small pixel value, and for example, the assay region image 62 shown as an example in Fig. 8 shows a pixel value of 130 or less. On the other hand, other portions of the assay region L1 show pixel values of 185 to 208.

As described above, in the image 61 of Fig. 8, the line-shaped assay region L1 is not expressed, but a high-density portion d is included in a part thereof. In such a case, in a case where the main determination is performed in a state where all the pixels composing the high-density portion d are included, in some case, the determination is made as positive due to the high-density portion d even though the sample is negative. The abnormality determination unit 127 determines the presence or absence of abnormality such as high-density portion d in which the main determination may cause an erroneous determination in such an assay region L1.

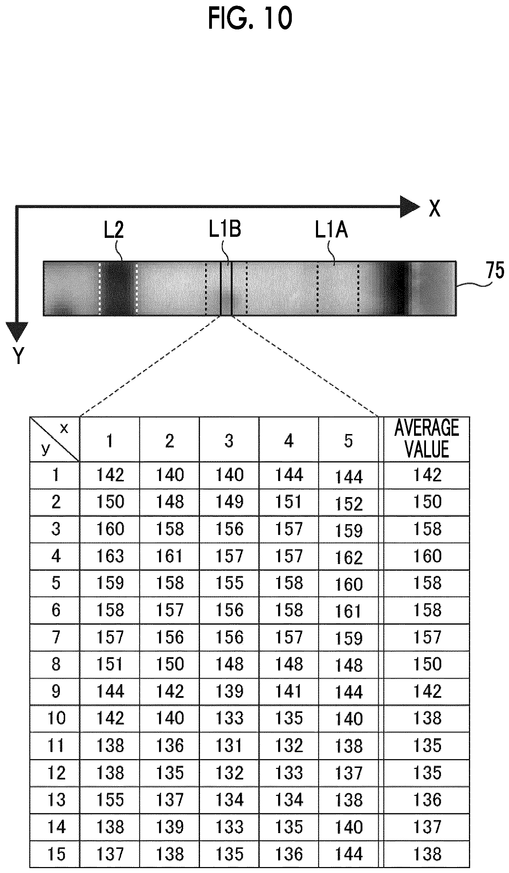

Next, a specific method of determining abnormality will be described. In Fig. 9, the observation image 74 shown in Fig. 9A and the observation image 75 shown in Fig. 9B (and Fig. 10) each are examples of observation images, Fig. 9C represents the displacement of the pixel value in one pixel sequence extracted from each of the first assay region L1A of the observation image 74 in Fig. 9A and the second assay regions L1B of the observation image 75 in Fig. 9B.

The observation image 61 is shown as a schematic diagram in Fig. 8, but the observation images 74 and 75 are shown as an actual image instead of the schematic diagram for convenience in Fig. 9. The observation image 61 shown in Fig. 8 and the observation images 74 and 75 shown in Fig. 9 are common in that both are images obtained by imaging the observation region, but in the observation region shown by the observation image 61 and the observation regions shown by the observation images 74 and 75, the configuration such as the assay region L1 and the like are different. That is, while the observation image 61 shown in Fig. 8 includes three regions of the assay region L1, the control region L2, and the color development region L3, the observation images 74 and 75 shown in Fig. 9 include two assay regions, the first assay region L1A and the second assay region L1B, and the control region L2. That is, the cartridge shown here is a cartridge for assaying the presence or absence of two types of test substances.

In a cartridge for assaying the presence or absence of two types of test substances, the two types of test substances are, for example, influenza A type and influenza B type. In Fig. 9A and Fig. 9B, the first assay region L1A is an assay region including a capture antibody for influenza A type, and the second assay region L1B is an assay region including a capture antibody for influenza B type. The width of the first assay region L1A, the second assay region L1B, and the control region L2 in the X direction is, for example, 1 mm, and the three regions of the first assay region L1A, the second assay region L1B, and the control region L3 are provided, for example, at 3 mm interval. Hereafter, in a case where it is necessary to distinguish a plurality of assay regions L1, a subdivided reference numeral of A or B is added to a reference numeral of the assay region L1, and in a case where it is not necessary to distinguish the plurality of assay regions L1, the reference numerals are simply referred to as assay regions L1.

In the observation image 74 of the example shown in Fig. 9A, the line of the first assay region L1A is expressed, and the line of the second assay region L1B is not expressed. On the other hand, in the observation image 75 shown in Fig. 9B, no line is expressed in both of the first assay region L1A and the second assay region L1B, but in the lower part of the second assay region L1B of the observation image 75, there is a partial high-density portion corresponding to stains. Such a state in which a partial high-density portion is generated in the assay region is a state in which abnormality is present in the assay region. The abnormality determination unit 127 determines such the presence or absence of the abnormality in the assay region, that is, the presence or absence of a partially occurring high-density portion.

As described above, the observation image 74 and the observation image 75 are images in which a plurality of pixels are two-dimensionally arranged in a matrix form. Fig. 9C shows the relationship between the pixel value of one pixel sequence a in the assay region image showing the first assay region L1A of Fig. 9A and the pixel position in the Y direction, and the relationship between the pixel value of one pixel sequence b in the assay region image showing the second assay region L1B of Fig. 9B and the pixel position in the Y direction.

As shown in Fig. 9C, the variation of the pixel values of the pixels included in the pixel sequence b of the second assay region L1B having abnormality in the observation image 75 is large as compared with the pixel values of the pixels included in the pixel sequence a of the first assay region L1A having no abnormality in the observation image 74. In a case where the sample 50 is positive and a normal line is expressed in the assay region L1, as shown in the pixel sequence a without abnormality, the density of the pixels in the assay region L1 increases uniformly, and thus there is no significant difference in the pixel values of the pixels arranged in the Y direction. Similarly, in a case where the sample 50 is negative and the line is not expressed in the assay region L1, the density of the pixels in the assay region L1 does not change, and thus there is no significant difference in the pixel values of the pixels arranged in the Y direction. On the other hand, the partial high-density portions generated by the nonspecific adsorption rarely occur uniformly in the Y direction, as shown in the pixel sequence b having the abnormality, variation of the pixel values of the pixels arranged in the Y direction (so-called dispersion in the pixel value) is larger as compared with a case where the high-density portion is not generated.

Therefore, it is possible to determine the presence or absence of abnormality in the assay region image using the magnitude of variation in the pixel values of the pixels arranged in the Y direction in the assay region image as a determination index. Examples of the scale for measuring the magnitude of variation in the pixel value include a change amount of a pixel, a standard deviation, and a variation coefficient in a pixel sequence. Here, the change amount of a pixel is a difference between a relatively high pixel value and a low pixel value in the pixel sequence, and, as an example, is a difference between a maximum value and a minimum value. However, the change amount used as the determination index may be a difference between a relatively high pixel value and a relatively low pixel value within a range in which a difference that can distinguish a case having an abnormality and a case having no abnormality is recognized. For example, a difference between the second largest pixel value and the second smallest pixel value in the pixel sequence, a difference between the third largest pixel value and the third smallest pixel value, or the like may be used as the change amount.

Table 1 shows results that the change amount (maximum value - minimum value), the standard deviation, and the variation coefficient each are calculated based on the pixel values, as shown in Fig. 9C, of the pixel composed of the pixel sequence b having an abnormality in the assay region L1 and the pixel composed of the pixel sequence a having no abnormality in the assay region.

**[Table 1]**

| | Abnormality is present | Abnormality is absent |
|---|---|---|
| Change amount (maximum value - minimum value) | 27 | 14 |
| Standard deviation | 10.3 | 4.4 |
| Variation coefficient | 7.1% | 3.0% |

As shown in Table 1, in the case of the pixel sequence b having an abnormality, the standard deviation, the change amount, and the variation coefficient are large as compared with those in the case of the pixel sequence a having no abnormality. Therefore, the presence or absence of an abnormality is determined using at least one of the standard deviation, the amount of change, or the variation coefficient as a determination index. As an example, in a case where the standard deviation is used as the determination index, for example, it is determined that abnormality is present in the standard deviation of 8 or more, and it is determined that abnormality is absent in the standard deviation of less than 8. Further, in a case where the change amount is used as the determination index, for example, it is determined that abnormality is present in the change amount of 20 or more, and it is determined that abnormality is absent in the change amount of less than 20. In a case where the variation coefficient is used as the determination index, for example, it is determined that abnormality is present in the variation coefficient of 5% or more, and it is determined that abnormality is absent in the variation coefficient of less than 5%. In addition, in a case where the standard deviation and the change amount are used as determination index, for example, it is determined that abnormality is present in the standard deviation of 8 or more and the change amount of 20 or more, and it is determined that abnormality is absent in the standard deviation of less than 8 or the change amount of less than 20. The numerical value that is a determination reference for the presence or absence of an abnormality (hereinafter, referred to as the determination reference value TA) is a value that is appropriately set according to the test substance as a target, the type of the cartridge, the assay apparatus, and the like, and the preset value is stored in the memory 121 as setting information.

It is described above that at least one of a change amount, a standard deviation, or a variation coefficient regarding a pixel value of a pixel included in one column (one pixel sequence) in an assay region image indicating the assay region L1 is used as a determination index. As the pixel sequence used for calculating the determination index, any one column in the assay region image may be extracted, and any one extracted column may be used for the abnormality determination. As the extraction position, it is preferable to extract one column in the vicinity of the center in the X direction in the assay region image. This is because, in many cases, the difference between the case where abnormality is present and the case where abnormality is absent is more remarkable in the vicinity of the center. In addition, the determination index is calculated for each of a plurality of columns in the assay region image without being limited to one column, and in a case where a determination reference value TA is satisfied for the determination indexes of all of the plurality of columns or a majority of the columns, it may be determined that abnormality is present.

Further, among the representative values of each row, which are derived based on a plurality of pixels present in the same row and in different columns in the assay region image, the processor 120 may use, as the determination index, at least one of a difference between the relatively large representative value and the relatively small representative value, or the standard deviation or the variation coefficient based on the representative value of each row. For example, as shown in Fig. 10, a plurality of columns are extracted from the assay region image indicating the second assay region L1B in the observation image 75, and the average value of the pixel values of a plurality of pixels present in the same row and in different columns is derived as a representative value. In Fig. 10, as an example, pixel sequences are extracted in five columns. Each column includes pixels for 15 rows. In Fig. 10, the numerical value shown in each pixel of the pixels arranged in a matrix form is a pixel value. The processor 120 derives, for example, an average value for each row of the pixel sequences of the five columns, and uses this average value as a representative value of each row. In the calculation using the example of the pixel value of Fig. 9, for example, the representative value of the first row is (142 + 140 + 144 + 144)/5 = 142. The representative values for the 15 rows derived in this manner are regarded as one column, and at least one of the change amount, the standard deviation, or the variation coefficient is derived in the same manner as described above and used as the determination index.

In this way, among the representative values of each row, which are derived based on a plurality of pixels present in the same row and in different columns, at least one of a difference between the relatively large representative value and the relatively small representative value, or the standard deviation or the variation coefficient based on the representative value of each row is used as the determination index, and thus noise is suppressed and the abnormality determination with high accuracy can be performed, as compared with a case where the determination index is obtained using one specific column of the assay region.

The above-described abnormality determination is performed by the method described above. The processor 120 needs to extract the assay region image from the observation images 74 and 75 (see Fig. 9) prior to the determination of the presence or absence of the abnormality in the assay region image corresponding to the assay region L1. Hereinafter, a method of extracting the assay region will be described.

For example, the processor 120 reads out the position information of the assay region L1 in the observation image previously held in the memory 121 as the setting information, specifies the assay region L1 in the observation images 74 and 75, and extracts the assay region image. The position information of the assay region L1 is, for example, a distance from one end of the observation image.

As described above, the detection image can be easily extracted by performing the extraction based on the position information. On the other hand, due to individual differences in the cartridge 100, slight misregistration may occur at the position of the assay region L1 in the observation images 74 and 75. Therefore, in a case where the assay region L1 is extracted from the observation images 74 and 75 based only on the position information, misregistration from the actual assay region L1 may occur. Therefore, it is preferable to derive a profile in the row direction based on the representative values of the respective columns in the observation images 74 and 75, which are images in which a plurality of pixels are two-dimensionally arranged in a matrix form, and extract the assay region image corresponding to the assay region from the observation image based on the derived profile.

Hereinafter, a method of extracting the detection region image using the profile will be described. The processor 120, first derives representative values of each of columns of the observation image. The method of deriving the representative value is not particularly limited, and for example, in each column, the pixel value of a specific row included in the column may be used as the representative value, or the average value or center value of the pixel values of two or more pixels included in the column may be used as a representative value.

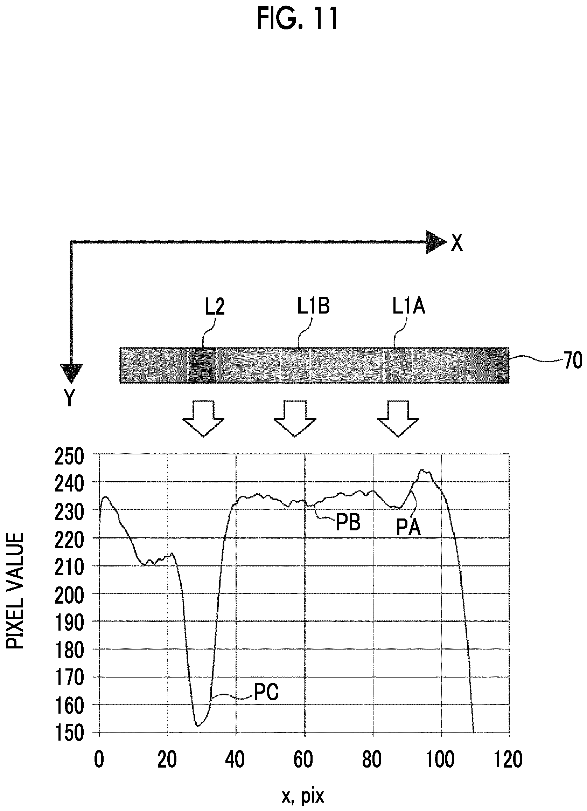

The processor 120 generates a profile of the pixel values in the row direction (that is, the X direction) using the derived representative values. Fig. 11 shows an example of a profile generated from the observation image 70. In the profile using the representative values of respective columns, the vertical axis represents the pixel value and the horizontal axis represents the pixel position in the X direction. Observation image 70 is an observation image in a case where a cartridge for an influenza test for assaying two types of test substances is used, similarly to the observation images 74 and 75 shown in Fig. 9A and Fig. 9B. Similarly to the observation images 74 and 75, the observation image 70 includes two assay regions L1A and L1B and a control region L2.

In the observation image 70 shown in Fig. 11, the control region L2 is clearly expressed in a line shape. In addition, the first assay region L1A is also expressed in a line shape, while it is thinner than the control region L2. On the other hand, the second assay region L1B is not expressed. Each region of PA, PB, and PC in the profile shown in Fig. 11 corresponds to each of the first assay region L1A, the second assay region L1B, and the control region L2.

The processor 120 extracts the assay region profile from the profile generated using the representative value. Specifically, the processor 120 extracts the assay region profile PA corresponding to the first assay region L1A and the assay region profile PB corresponding to the second assay region L1B from the profile of Fig. 11. In a case of extracting the assay region profile, the first assay region L1A and the second assay region L1B may be extracted after converting the profile of the pixel value into the density profile. Since a portion having a high density, which is a bottom in the profile of the pixel value, shows a peak in the density profile, the first assay region L1A and the second assay region L1B can be extracted by extracting the peak from the density profile. For example, the processor 120 specifies the position of the first assay region L1A by collating the position information of the first assay region L1A with the position of the bottom of the assay region profile PA shown in Fig. 11 (the peak position in the case of the density profile). Then, the processor 120 extracts the assay region image from the position specified from the observation image 70. The position information of the first assay region L1A and the second assay region L1B in the observation image 70 is, for example, stored in advance in the memory 121 as setting information. The setting information is, for example, a distance from one end of the observation image 70.

As described above, the processor 120 generates a profile of the pixel value (or a density profile), and the position of the assay region L1 in the observation image is specified by collating the position of the bottom (or peak) corresponding to the assay region L1 extracted from the generated profile with the position information of the assay region L1. Therefore, it is possible to more accurately extract the assay region profile of the assay region L1 as compared with the case where only one of the position of the bottom of the profile and the position information is used. In this way, since the abnormality determination is performed using the assay region images extracted from the observation images 74 and 75 with high position accuracy, the accuracy of the abnormality determination can be improved.

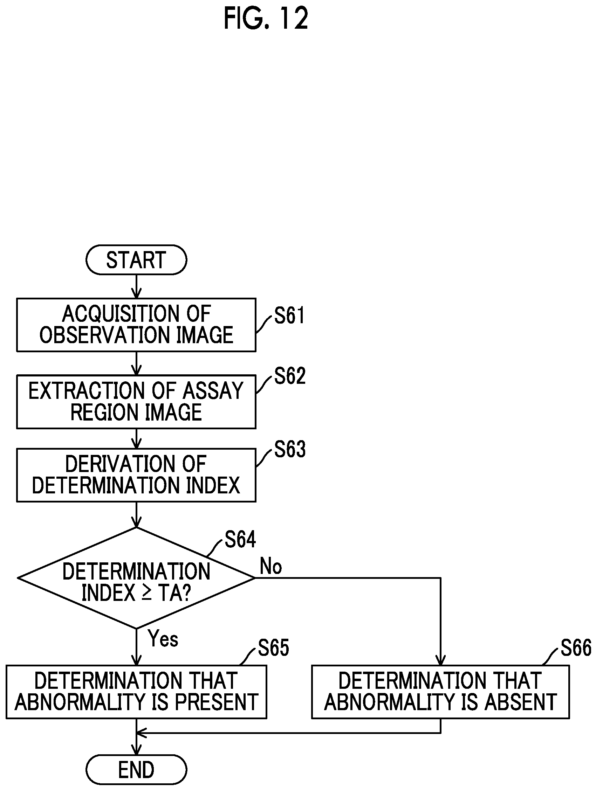

Fig. 12 shows a processing procedure of abnormality determination by the processor 120.

In the processing procedure of abnormality determination, the processor 120 first acquires the observation image 74 (or the observation image 75) by cutting out the observation region from the captured image output by the imaging unit 114 (Step S61).

Next, the processor 120 extracts the assay region image from the acquired observation image 74 (or the acquired observation image 75) (Step S62). A method of extracting an assay region image is as described.

The processor 120 derives a determination index from the extracted assay region image (Step S63). As described above, for example, the processor 120 derives, as a determination index, at least one of the change amount, the standard deviation, and the variation coefficient regarding a pixel value of a pixel included in at least one column (one pixel sequence) in the assay region image. Alternatively, among the representative values of each row, which are derived based on a plurality of pixels present in the same row and in different columns in the assay region image, the processor 120 derives, as the determination index, at least one of a difference between the relatively large representative value and the relatively small representative value, or the standard deviation or the variation coefficient based on the representative value of each row.

The processor 120 determines whether or not the derived determination index is not less than the preset determination reference value TA (Step S64). In a case where the determination index is not less than the determination reference value TA (Step S64: Yes), the processor 120 determines that abnormality is present (Step S65), and ends the processing of the abnormality determination. On the other hand, in a case where the determination index is less than the determination reference value TA (Step S64: No), the processor 120 determines that abnormality is absent (Step S66), and ends the processing of the abnormality determination. The processing procedure of the abnormality determination by the processor 120 is as described above.

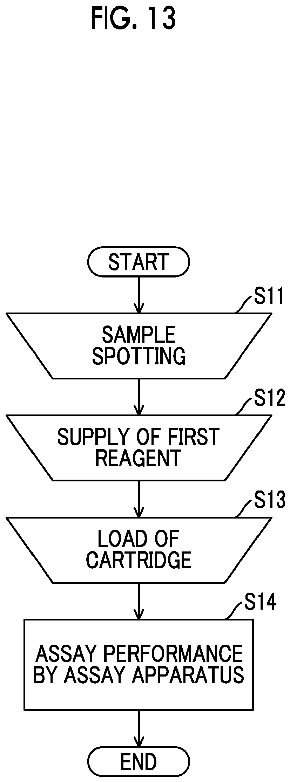

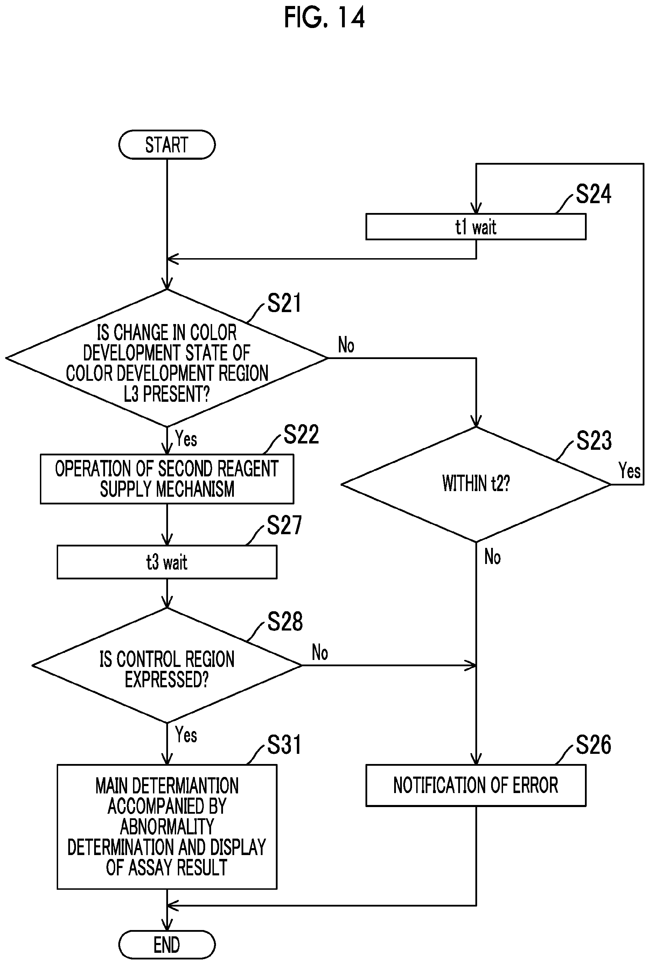

The procedure of the immunochromatographic assay using the assay apparatus 110 of the present embodiment will be described with reference to Fig. 13 and Fig. 14. Here, a first assay flow that is an aspect in which the supply of the first reagent 41 is performed by the user and the supply of the second reagent 46 is performed by the assay apparatus 110 will be described.

### (First assay flow)

Fig. 13 is a diagram showing the first assay flow.

First, a user adds dropwise the sample 50 from the dropping port 16 of the cartridge 100 onto the spotting region of the carrier 2 (Step S11).

Next, the user presses the first pressed part 11 of the cartridge 100 to start the supply of the first reagent 41 (Step S12).

After that, the user loads the cartridge 100 into the loading part 112 of the assay apparatus 110 in a state where a power is turned on (Step S13).

An assay of the loaded cartridge 100 is performed in the assay apparatus 110 (Step S14).

The time from the start of the supply of the first reagent 41 until the first reagent 41 is sufficiently developed in the carrier 2 varies depending on each cartridge, but it takes generally about 5 minutes to 10 minutes. The time from the pressing the first pressed part 11 by the user until the first pressed part 11 is loaded into the loading part 112 may be set according to the convenience of the user.

Fig. 14 shows a detailed assay flow of an assay performance (Step S14) by the assay apparatus 110 shown in Fig. 13.

By loading the cartridge 100 into the assay apparatus 110, the assay in the assay apparatus 110 (Step S14 in Fig. 13) is started.

As shown in Fig. 14, in the assay apparatus 110, first, the processor 120 discriminates whether or not the color development state of the color development region L3 is changed (specifically, the change from a dark green color to an orange color) (Step S21). Specifically, the processor 120 illuminates the observation region 68 exposed from the observation window 18 by turning on the light source 115, and causes the imaging unit 114 to perform imaging in that state. Then, the processor 120 acquires the captured image from the imaging unit 114, and discriminates the change in the color development state of the color development region L3 from the acquired captured image. In a case where it is discriminated that a change in the color development state of the color development region L3 is present, that is, in a case where the color of the color development region L3 has changed from a dark green color to an orange color, it means that the first reagent 41 has reached the color development region L3, and the assay region L1 and the control region L2 which are on the upstream side of the color development region L3.

In a case where the color development state of the color development region L3 has changed (Step S21: Yes), the processor 120 operates the second reagent supply mechanism 118 to start the supply of the second reagent 46 (Step S22). In the present embodiment, the processor 120 presses the second pressed part 12 of the cartridge 100 by the second reagent supply mechanism 118. In a case where the second pressed part 12 is pressed, the second pressed part 12 is deformed to sink toward the second reagent holding part 45. Due to this deformation, the sheet member 48 of the second reagent holding part 45 is pressed against the protrusion 34 to break, and the second reagent 46 is supplied onto the carrier 2.

On the other hand, in a case where the color development state of the color development region L3 has not changed (Step S21: No), the determination of whether or not the it is within the second setting time t2 is performed (Step S23).

Here, in a case where the process has exceeded the second setting time t2 (Step S23: No), the processor 120 notifies an error (Step S26) and ends the assay flow. For example, the notification of the error is performed by displaying an error message on the monitor 119. In addition to displaying the error message on the monitor 119, as a method of notifying the error, the error message may be notified by voice.

On the other hand, in a case where the it is within the second setting time t2 (Step S23: Yes), the process waits until the first setting time t1 elapses (Step S24). In Step S24 of Fig. 14, it is shown as "t1 wait". The first setting time t1 is, for example, about 30 seconds, and the second setting time t2 is preset to, for example, 20 minutes or the like. After that, the process returns to Step S21 of discriminating whether or not the color development state of the color development region L3 has changed.

In Step S22, after the second reagent supply mechanism 118 is operated to start the supply of the second reagent 46, the process waits until a preset third setting time t3 elapses (Step S27). In Step S27 of Fig. 14, it is shown as "t3 wait". The third setting time t3 is, for example, about 3 minutes.

After that, the processor 120 discriminates whether or not the line is expressed in the control region L2 (Step S28). Specifically, similarly to Step S21, the processor 120 causes the imaging unit 114 to image the observation region 68 in a state where the observation region 68 is illuminated. Then, the processor 120 acquires the captured image from the imaging unit 114, and cuts out an observation image 61 of the observation region 68 from the acquired captured image. Then, the processor 120 discriminates the change in the color development state of the control region L2 of the cut-out observation image 61. The processor 120 discriminates, for example, whether or not the color optical density of the control region L2 reaches a density not less than a preset reference, and in a case where the density is not less than the reference, the processor 120 discriminates that the control region L2 is expressed. The case where it is discriminated that the control region L2 is expressed means that the sample 50 has reached the control region L2 and the assay region L1 on the upstream side thereof, and are subjected to silver amplification.

In the discrimination of whether or not the control region L2 is expressed in Step S28, in a case where the control region L2 is not expressed (Step S28: No), an error is notified (Step S26) and the assay flow ends. In a case where the control region L2 is not expressed after the development of the second reagent 46, there is a possibility that the sample 50 has not been spotted.

On the other hand, in the discrimination of whether or not the control region L2 is expressed (Step S28), in a case where the control region L2 is expressed (Step S28: Yes), the processor 120 performs the main determination accompanied by the abnormality determination, and presents the assay result based on the result of the main determination accompanied by the abnormality determination (Step S31).

In Step S31, the processor 120 determines whether the sample 50 is positive or negative as the main determination. Specifically, the processor 120 discriminates whether the color optical density of the line-shaped assay region L1 reaches a density not less than a preset reference, and in a case where the density is not less than the reference, the processor 120 discriminates that the assay region L1 is expressed. In the case where it is discriminated that the assay region L1 is expressed, it is determined that the sample 50 is positive, and in the case where it is discriminated that the assay region L1 is not expressed, it is determined that the sample 50 is negative. In addition, as the abnormality determination, the processor 120 performs processing according to the processing procedure of abnormality determination shown in Fig. 12 to determine the presence or absence of abnormality in the assay region image. In Step S31, in the main determination accompanied by the abnormality determination, the processor 120 may perform the abnormality determination before the main determination or may perform the abnormality determination after the main determination. Therefore, a plurality of processing procedures can be considered as the processing procedure in Step S31. Hereinafter, the details of Step S31 will be described. In addition, hereinafter, five processing procedures of Step S31 will be exemplified, and the five processing procedures will be referred to as a first example to a fifth example to distinguish these.

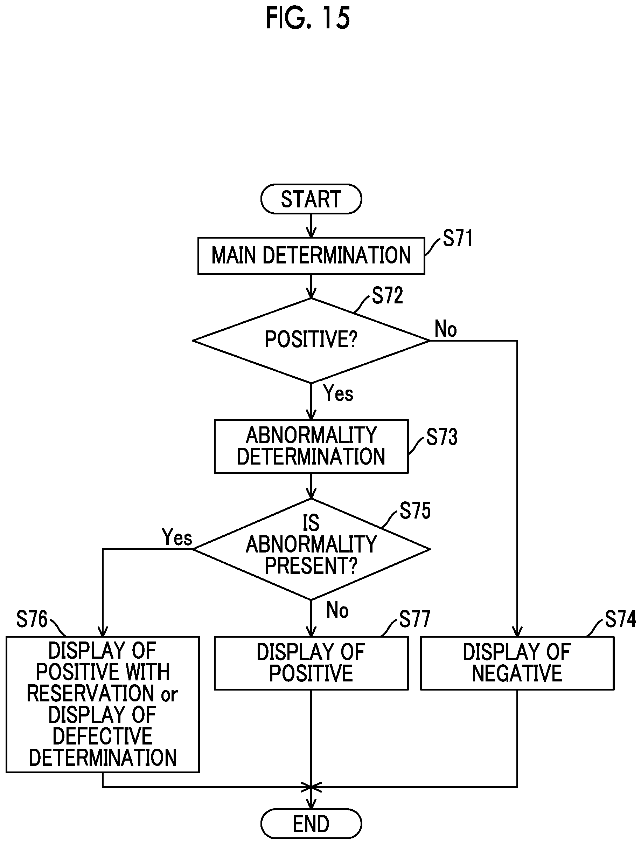

Fig. 15 shows a first example, which is an example of the processing procedure of Step S31.

As shown in Fig. 15, in the first example of Step S31, the processor 120 first performs the main determination of whether the sample 50 is positive or negative (Step S71).

In a case where it is determined that the result of the main determination is negative (Step S72: No), the processor 120 displays "negative" as the assay result on the monitor 119 without performing the abnormality determination, and ends the processing of Step S31 in Fig. 14. On the other hand, in a case where it is determined that the result of the main determination is positive (Step S72: Yes), the processor 120 performs the abnormality determination (Step S73).

In Step S73 as the abnormality determination, the processor 120 performs the abnormality determination according to the processing procedure of the abnormality determination shown in Fig. 12, using the assay region image extracted from the observation image acquired in the step of the main determination.

In a case where the processor 120 determines that abnormality is present as a result of the abnormality determination (Step S75: Yes), the processor 120 displays "positive with reservation" as the assay result on the monitor 119 (Step S76), and ends the processing of Step S31 in Fig. 14. Here, "with reservation" means that reservation indicating that abnormality is present in the assay region image is included. That is, the display indicating positive with reservation is to make the user recognize that there is a possibility of false positive because abnormality is present in the assay region L1, while the result of the main determination is positive.

In Step S76, as the assay result, instead of displaying the determination result that is positive with reservation, an error message of "defective determination" may be displayed without displaying the determination result. In addition to the "defective determination", it may be presented that the assay region may be stained. In a case where the assay result that is positive with reservation or defective determination is displayed, the user can judge to confirm whether the sample is positive or negative by visually discriminating whether the presence or absence of the color development state of the assay region, to be re-assayed, or the like.

On the other hand, in a case where it is determined that the result of the abnormality determination is no abnormality (Step S75: No), the processor 120 displays "positive" as the assay result on the monitor 119 (Step S77) and ends the processing of Step S31 in Fig. 14.

In the present assay apparatus 110, the processor 120 performs the main determination accompanied by the abnormality determination according to the above-described processing procedure of the abnormality determination. That is, the processor 120 performs abnormality determination using, as a determination index, at least one of a difference between a relatively large pixel value and a relatively small pixel value in at least one column in the assay region image, or a standard deviation or a variation coefficient of the pixel values of the pixels included in the at least one column. Alternatively, among the representative values of each row, which are derived based on a plurality of pixels present in the same row and in different columns in the assay region image, the processor 120 performs abnormality determination using, as the determination index, at least one of a difference between the relatively large representative value and the relatively small representative value, or the standard deviation or the variation coefficient based on the representative value of each row. Then, the processor 120 determines the processing content related to the main determination based on the presence or absence of the abnormality. Therefore, the presence or absence of an abnormality in the assay region L1 can be determined with high accuracy, and the processing content related to the main determination is determined based on the presence or absence of the abnormality and thus it is possible to present an assay result with higher reliability.

In particular, in the first example of Step S31 described above, the processor 120 first performs the main determination, and performs the abnormality determination in a case where the determination result of the main determination is positive. Based on the presence or absence of abnormality, the method of presenting the result of main determination as the processing content related to the main determination is made different. That is, in a case where the result of main determination is positive, the determination result of the main determination is presented as the assay result as it is (here, "positive" is displayed on the monitor 119) in a case where abnormality is absent, or the determination result of the main determination is presented with reservation indicating that abnormality is present in the assay region image (here, "positive with reservation" is displayed on the monitor 119) in a case where abnormality is present. Accordingly, it is possible to present to the user whether the determination result has sufficiently high reliability or includes a possibility of having a possibility of false positive, and the reliability of the assay result can be improved.

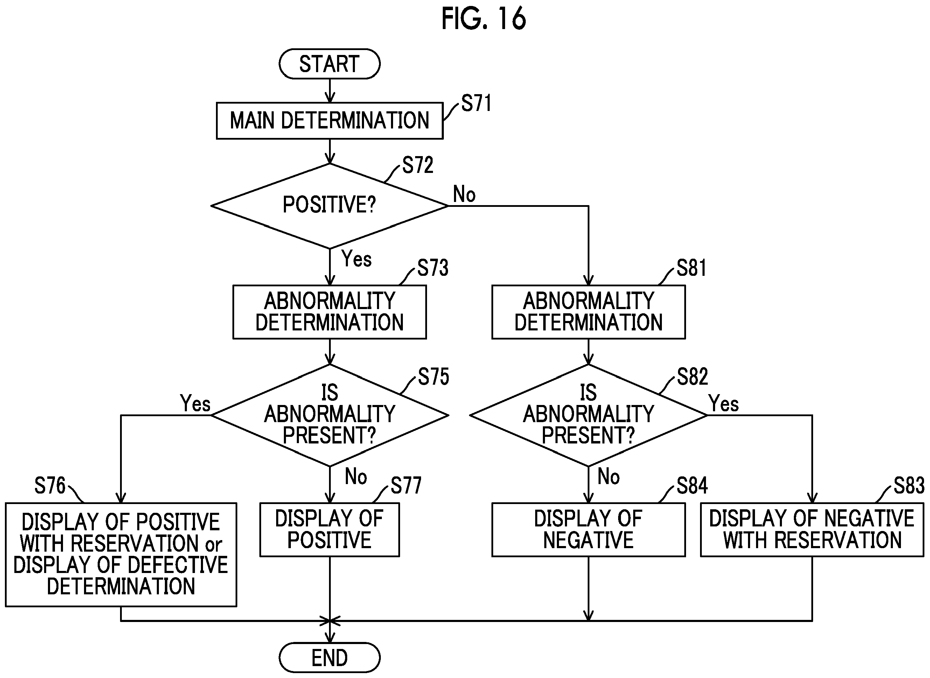

Fig. 16 shows a second example of the processing procedure of Step S31. In Fig. 16, the same steps as those in the first example shown in Fig. 15 is denoted by the same reference numeral for step, and detailed description thereof will be omitted.

In the second example of Step S31, in the case where the processor 120 performs the main determination (Step S71) and determines that the sample is negative (Step S72: No), the processing thereafter is different from that of the second example. In the second example, also in a case where the processor 120 performs the main determination (Step S71) and determines that the sample is negative (Step S72: No), the abnormality determination is performed (Step S81). Then, in a case where it is determined that abnormality is present as the result of the abnormality determination in Step S81 (Step S82: Yes), the processor 120 displays "negative with reservation" on the monitor 119 (Step S83) and ends that processing of Step S31 in Fig. 14.

On the other hand, in a case where it is determined that abnormality is absent as the result of the abnormality determination in Step S81 (Step S82: No), the processor 120 displays "negative" on the monitor 119 (Step S84) and ends that processing of Step S31 in Fig. 14.

Also in the second example shown in Fig. 16, since the abnormality determination is performed, the presence or absence of abnormality in the assay region image is determined, and the processing content related to the main determination is determined based on the presence or absence of the abnormality, it is possible to present an assay result with higher reliability.

In particular, in the second example shown in Fig. 16, in a case where it is determined that abnormality is absence (Step S75: No and Step S82: No), the processor 120 presents the determination result of the main determination as the assay result as it is (here, "positive" or "negative" is displayed on the monitor 119), and in a case where it is determined that abnormality is present (Step S75: Yes and Step S82: Yes), the processor 120 presents the determination result of the main determination with reservation indicating that the abnormality is present in the assay region image (here, "positive with reservation" or "negative with reservation" is displayed on the monitor 119). Accordingly, it is possible to present to the user whether the assay result has sufficiently high reliability or includes a possibility of having a possibility of false positive, and the reliability of the assay result can be improved.

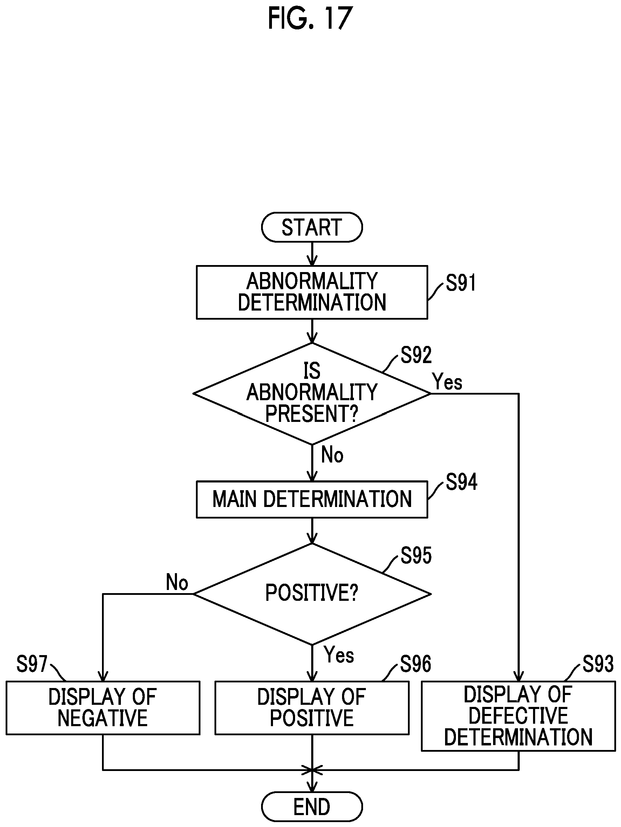

In the first example shown in Fig. 15 and the second example shown in Fig. 16, the main determination is performed first, and then the abnormality determination is performed. However, in Step S31, the abnormality determination may be performed followed by the main determination. Fig. 17 is yet another example showing a detailed processing procedure of Step S31, and shows a third example in a case where the abnormality determination is performed followed by the main determination.

As shown in Fig. 17, in the third example, the processor 120 first performs the abnormality determination (Step S91). The processor 120 performs processing according to the processing procedure of abnormality determination shown in Fig. 12.

In a case where it is determined that abnormality is present in the abnormality determination (Step S92: Yes), the processor 120 displays "defective determination" on the monitor 119 without performing the main determination (Step S93), and ends the processing of Step S31 in Fig. 14. At this time, instead of or together with the display of the defective determination, a description such as "assay region is stained and main determination is not performed" may be displayed on the monitor 119.

In a case where it is determined that abnormality is absent in the abnormality determination (Step S92: No), the processor 120 performs the main determination (Step S94). In a case where the main determination is performed after the abnormality determination is performed, the observation image acquired in Step S91 of the abnormality determination is used.

In a case where it is determined that the sample is positive as a result of the main determination (Step S95: Yes), the processor 120 displays "positive" on the monitor 119 (Step S96), and ends the processing of Step S31 in Fig. 14. On the other hand, in a case where it is determined that the sample is negative as a result of the main determination (Step S95: No), the processor 120 displays "negative" on the monitor 119 (Step S97), and ends the processing of Step S31 in Fig. 14.

In the third example shown in Fig. 17, since the main determination is performed after performing the abnormality determination and confirming that abnormality is absent, the reliability of the assay result can be improved. Furthermore, in the third example, in a case where it is determined that abnormality is present in the assay region image, the assay ends without occupying the assay apparatus 110 any more, and thus the occupancy time of the assay apparatus 110 can be shortened.

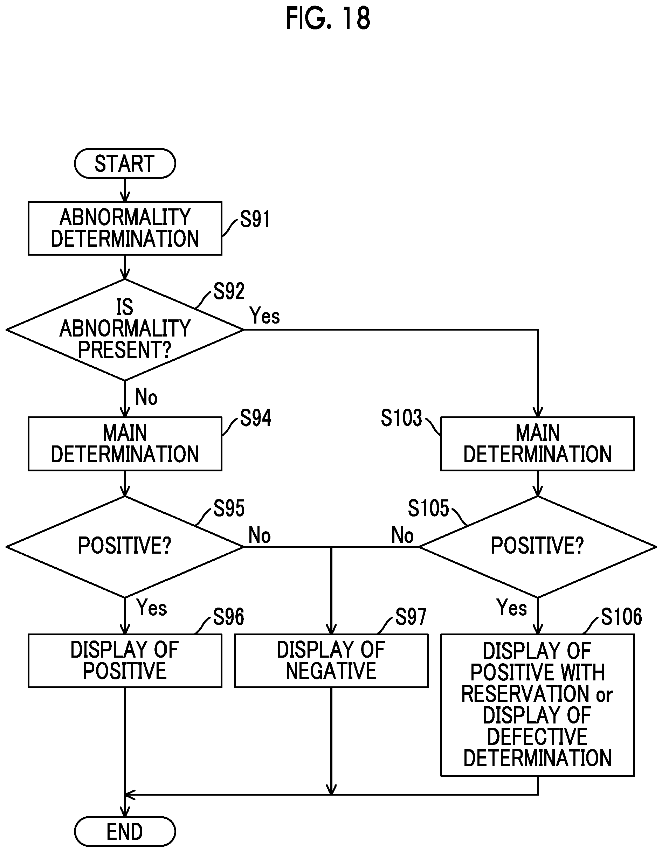

In a case where the main determination is performed after the abnormality determination is performed as in the third example shown in Fig. 17, the main determination may be performed even in a case where it is determined that abnormality is present in the abnormality determination. Fig. 18 is an example showing a detailed processing procedure of Step S31, and shows a fourth example in a case where the abnormality determination is performed first and it is determined that abnormality is present. In Fig. 18, the same steps as those in the third example shown in Fig. 17 are denoted by the same reference numeral for step, and detailed description thereof will be omitted.

As shown in Fig. 18, in the fourth example, the processor 120 performs the abnormality determination (Step S91), and performs the main determination also in a case where it is determined that abnormality is present (Step S92: Yes) (Step S103). Then, in a case where it is determined that the sample is positive as a result of the main determination of Step S103 (Step S105: Yes), the processor 120 displays "positive with reservation" on the monitor 119. On the other hand, in a case where it is determined that the sample is negative as a result of the main determination of Step S103 (Step S105: No), the processor 120 displays "negative" on the monitor 119. Other steps are the same as in the third example.

In a case where it is determined that the sample is positive as a result of the main determination of Step S103 (step S105: Yes), the processor 120 may present an error message on the monitor 119 (here, "defective determination" is displayed on the monitor 119) instead of displaying "positive with reservation" (Step S106).

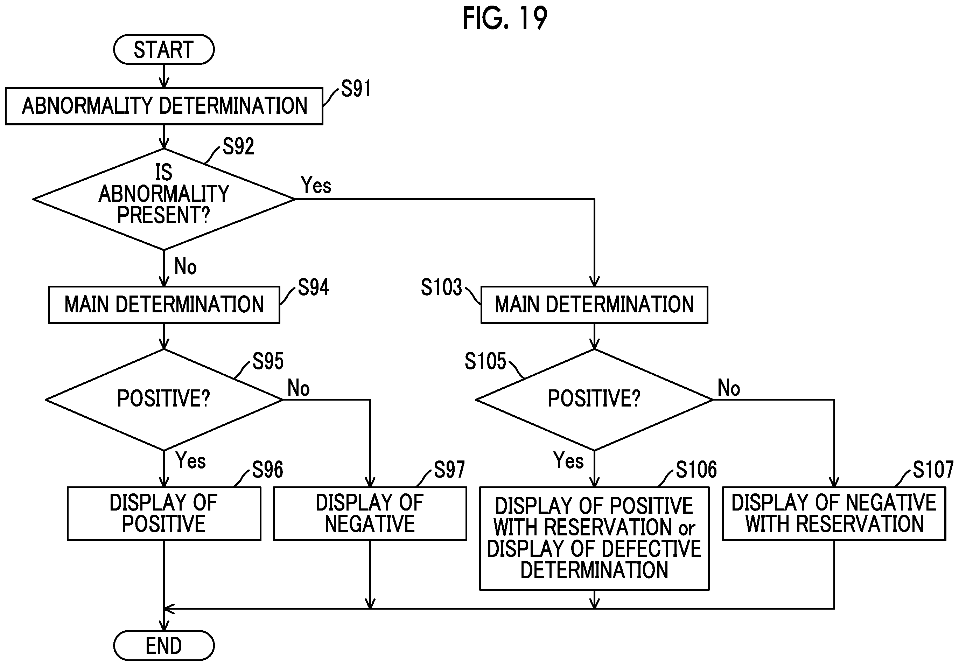

Fig. 19 is an example showing a detailed processing procedure of Step S31, and shows a fifth example in a case where the abnormality determination is performed first and it is determined that abnormality is present. In Fig. 19, the same steps as those in the third example or the fourth example are denoted by the same reference numeral for step, and detailed description thereof will be omitted.

As shown in Fig. 19, in the fifth example, the processor 120 performs the abnormality determination (Step S91), and performs the main determination also in a case where it is determined that abnormality is present (Step S92: Yes) (Step S103). Then, in a case where it is determined that the sample is negative as a result of the main determination of Step S103 (Step S105: No), the processor 120 displays "negative with reservation" on the monitor 119 (Step S107). Other steps are the same as in the fourth example.

Also in the fourth example shown in Fig. 18 and the fifth example shown in Fig. 19, similarly to the third example shown in Fig. 17, since the main determination is performed after confirming that abnormality is absent by the abnormality determination, the reliability of the assay result can be improved.

In the first example to the fifth example described above, in the case where the determination result of the defective determination or the determination result with reservation is presented as the assay result, the user can take measures to visually confirm whether or not the line of the assay region L1 is expressed, to perform treatment to be re-assayed, or the like.

In the above-described embodiment, as the processor 120 and a hardware structure of a processing unit as internal configurations thereof that executes various types of processing, such as a detection unit control unit 122, a color development state discrimination unit 123, a first reagent supply mechanism control unit 124, a second reagent supply mechanism control unit 125, an abnormality determination unit 127, and a display control unit 126, various processors shown below can be used. The various processors include, for example, a CPU which is a general-purpose processor executing software to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors, or may be configured by a combination of two or more processors having the same type or different types (for example, a combination of a plurality of FPGAs and/or a combination of a CPU and an FPGA). In addition, a plurality of processing units may be formed of one processor.

As an example in which a plurality of processing units are configured into a single processor, there is a form in which a single processor is configured by a combination of one or more CPUs and software, and this processor functions as a plurality of processing units. A second example of the configuration is an aspect in which a processor that implements the functions of the entire system including a plurality of processing units using one integrated circuit (IC) chip is used. A representative example of this aspect is a system on chip (SoC). In this way, various processing units are configured by one or more of the above-described various processors as hardware structures.

Furthermore, specifically, an electric circuit (circuitry) obtained by combining circuit elements, such as semiconductor elements, can be used as the hardware structure of the various processors.

The disclosure of Japanese patent application 2021-050780 filed on March 24, 2021 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. An immunochromatographic assay apparatus comprising:
a loading part in which a cartridge including a carrier having a spotting region on which a sample is spotted and an assay region in which a color development state changes depending on whether the sample is positive or negative, is attachably and detachably loaded;
an imaging unit that images the assay region; and
a processor configured to perform a main determination, which is a determination of whether the sample is positive or negative, based on an assay region image of the assay region imaged by the imaging unit,
wherein in a case where a development direction of the sample in the carrier is a row direction and a direction intersecting the row direction is a column direction, the assay region is a line-shaped region extending along the column direction,
the assay region image is an image in which a plurality of pixels are two-dimensionally arranged in a matrix form,
the processor is configured to perform an abnormality determination for determining presence or absence of an abnormality in the assay region image, using, as a determination index, at least one of a difference between a relatively large pixel value and a relatively small pixel value in at least one column in the assay region image, a standard deviation based on pixel values in each of the columns, or a variation coefficient based on pixel values in each of the columns, or at least one of a difference between a relatively large representative value and a relatively small representative value among representative values of all rows, which is derived based on a plurality of pixels existing in different columns in the same row in the assay region image, a standard deviation based on the representative values of all the rows, or a variation coefficient based on the representative values of all the rows, and
determine a processing content related to the main determination based on the presence or absence of the abnormality.

2. The immunochromatographic assay apparatus according to claim 1,
wherein the processing content includes any one of whether or not to perform the main determination or a method of presenting a main determination result of the main determination.

3. The immunochromatographic assay apparatus according to claim 2,
wherein the processor is configured to perform the main determination to present the main determination result in a case where the abnormality is absent, or not to perform the main determination in a case where the abnormality is present.

4. The immunochromatographic assay apparatus according to claim 3,
wherein the processor is configured to present that the main determination is not performed in a case where the main determination is not performed.

5. The immunochromatographic assay apparatus according to claim 3 or 4,
wherein the processor is configured to present that the assay region may be stained in a case where the abnormality is present.

6. The immunochromatographic assay apparatus according to claim 2,
wherein the processor is configured to present a determination result of the main determination in a case where the abnormality is absent, and present the determination result of the main determination with reservation indicating that the assay region image has an abnormality or not present the determination result of the determination in a case where the abnormality is present and in a case where the determination result of the main determination is positive.

7. The immunochromatographic assay apparatus according to claim 6,
wherein the processor is configured to perform the main determination, and perform the abnormality determination in a case where a determination result is positive.

8. The immunochromatographic assay apparatus according to claim 2,
wherein the processor is configured to present a determination result of the main determination in a case where the abnormality is absent, or present the determination result of the main determination with reservation indicating that the assay region image has an abnormality or not present the determination result in a case where the abnormality is present.

9. The immunochromatographic assay apparatus according to any one of claims 1 to 8,
wherein the imaging unit is an image sensor that images an observation region including the assay region to output an observation image including the observation region,
the observation image is an image in which a plurality of pixels are two-dimensionally arranged in a matrix form, and
the processor is configured to derive a profile in the row direction based on the representative values of the respective columns in the observation image, and extract the assay region image corresponding to the assay region from the observation image based on the derived profile.
